# EUROPEAN PATENT SPECIFICATION

(11) **EP 4 117 671 B1**
(45) Date of publication and mention of the grant of the patent: **18.11.2026**
(21) Application number: 21768205.3
(22) Date of filing: 10.03.2021
(51) Int. Cl.: C07C 237/26, A61K 31/65, A61P 25/32, A61P 31/00, C07C 237/52

(54) **NOVEL MODIFIED TETRACYCLINES FOR TREATMENT OF ALCOHOL USE DISORDER, PAIN AND OTHER DISORDERS INVOLVING POTENTIAL INFLAMMATORY PROCESSES**
NEUE MODIFIZIERTE TETRACYCLINE ZUR BEHANDLUNG VON ALKOHOLMISSBRAUCH, SCHMERZEN UND ANDEREN ERKRANKUNGEN MIT POTENZIELLEN ENTZÜNDLICHEN PROZESSEN
NOUVELLES TÉTRACYCLINES MODIFIÉES POUR LE TRAITEMENT DU TROUBLE D'UTILISATION D'ALCOOL, DE LA DOULEUR ET D'AUTRES TROUBLES IMPLIQUANT DES PROCESSUS INFLAMMATOIRES POTENTIELS

(30) Priority: 10.03.2020 US 202062987700 P
(43) Date of publication of application: 18.01.2023
(73) Proprietor: Texas Tech University System, Lubbock, TX 79409-2007 (US)
(72) Inventor: BERGESON, Susan, Lubbock, TX 79423 (US); SYAPIN, Peter, Camarillo, CA 93010 (US); REID, Ted W., Wolfforth Texas 79382 (US); SHASHTRI, Mayank, Lubbock, TX 79423 (US); TRAN, Phat, Lubbock, TX 79423 (US)
(74) Representative: SONN Patentanwälte GmbH & Co KG
(86) International application number: PCT/US2021/021679
(87) International publication number: WO 2021/183628

(56) References cited:
- WO-A1-2005/082860
- WO-A1-2019/241466
- WO-A1-2019/241490
- WO-A1-2019/241490
- US-A1- 2015 105 355
- US-A1- 2017 144 979
- US-A1- 2018 016 225

## Description

### TECHNICAL FIELD OF THE INVENTION

The present invention relates in general to the field of novel tetracycline derivatives with reduced antimicrobial activity for use in treating disorders of the central nervous system.

### BACKGROUND OF THE INVENTION

Without limiting the scope of the invention, its background is described in connection with modified tetracyclines.

Alcohol use disorder (AUD) or alcoholism is a condition that affects roughly 5% of individuals worldwide. AUD is characterized by an increased tolerance to alcohol and a physical dependence on alcohol making it hard for an individual to control intake. Some of the long-term effects of ingesting ethanol include cognitive and psychological changes, liver cirrhosis, gastritis, cardiomyopathy, anemia, and certain types of cancers. Alcoholism is caused by a complex mixture of genetic and environmental factors. There have been several genes linked to the way people metabolize alcohol and the development of AUD. The availability of alcohol also contributed to the number of people with AUD. Alcohol is the most available and widely abused recreational drug with beer being the third-most popular drink behind water and tea. There are currently very few methods for treating alcoholism outside of rehabilitation therapy which can be costly and very public. There is an unsatisfied need for a pharmaceutical component that is able to help combat the debilitating effects of AUD.

One such modified tetracycline derivative is taught in U.S. Patent Publication No. 20100173991, filed by Lorenz, et al., and entitled "Method for the synthesis of A-ring aromatized acetyl minocyclines". Briefly, these applicants are said to teach a less complex method for the production of A-ring aromatized acetyl minocyclines of the formula: 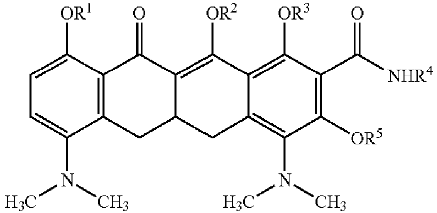 wherein R1 to R5 are acetyl and/or H, in which minocycline hydrochloride is reacted with acetic anhydride in the presence of a proton catcher and the reaction product is subjected to chromatographic filtration using a carrier material and an eluant. The eluant is distilled off, and the product is subsequently cleaned by recrystallization. However, this application is silent on the treatment of dependency disorders and only teaches the treatment of neurodegeneration.

Another such modified tetracycline derivative is taught in Patent Publication No. WO2009012741, also filed by Lorenz, et al., entitled "Method for the synthesis of A-ring aromatized acetyl minocyclines". The application is said to teach a method for the production of A-ring aromatized acetyl minocyclines that is less complex. The method for the production of A-ring aromatized acetyl minocyclines of the formula (I), wherein R1 to R5 = acetyl and/or H is achieved when minocycline hydrochloride is reacted with acetic anhydride in the presence of a proton catcher, the reaction product is subjected to chromatographic filtration using a carrier material and an eluant. The eluant is then distilled off, and the product is subsequently cleaned by recrystallization. Like the application described hereinabove, this application is also silent on the treatment of dependency disorders and only teaches the treatment of neurodegeneration.

WO 2019/241490 A1 discloses modified tetracyclines for treatment of alcohol use disorder, pain and other disorders involving inflammatory processes.

However, a need remains for novel molecules for the treatment of alcohol consumption, including, but not limited to high drinking levels, withdrawal symptoms and increased sensitization and duration of pain, and alter innate immune responses and reduce tobacco consumption and all aspects of the addiction process of other drugs subject to abuse, including opioids (Mark R. Hutchinson, Alexis L. Northcutt, Lindsey W. Chao, Jeffrey J. Kearney, Yingning Zhang, Debra L. Berkelhammer, Lisa C. Loram, Robert R. Rozeske, Sondra T. Bland, Steven F. Maier, Todd T. Gleeson, and Linda R. Watkins, Minocycline suppresses morphine-induced respiratory depression, suppresses morphine-induced reward, and enhances systemic morphine-induced analgesia, Brain, Behavior and Immunity 22(8): 1248-1256, 2009).

### SUMMARY OF THE INVENTION

The present invention is defined in the claims.

Disclosed is a modified tetracycline molecule, pharmaceutically acceptable salts, pro-drugs, biologically active metabolites, and tautomers thereof of comprising: a Deamino Diacetyl Minocycline, Methyl Ether Minocycline, Ethyl Ether Minocycline, Propyl Ether Minocycline, Butyl Ether Minocycline, Butyl Ether Monoacetyl Minocycline, Butyl Ether Diacetyl Minocycline, Buty Ether Triacetyl Minocycline, or Butyl Ether Tetra Acetyl Minocycline. The invention includes a modified tetracycline molecule, pharmaceutically acceptable salts, and tautomers thereof selected from: 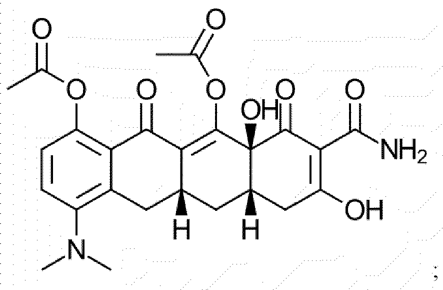 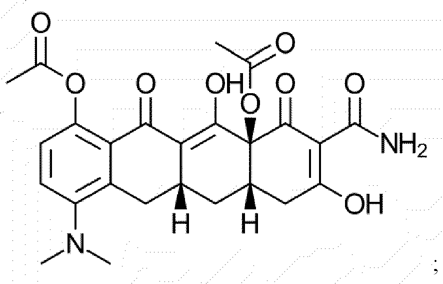 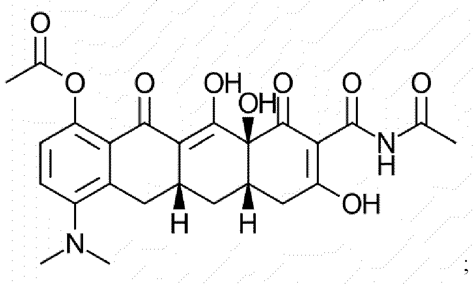 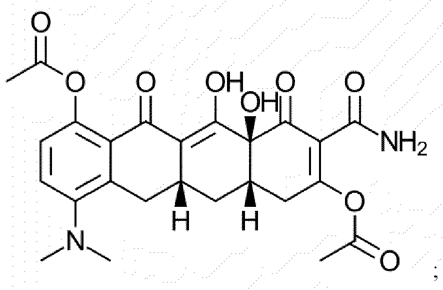 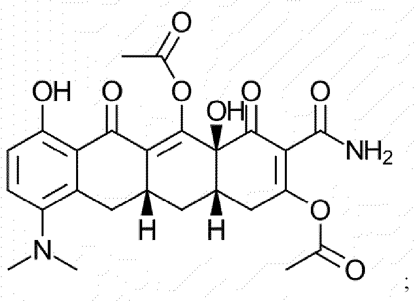 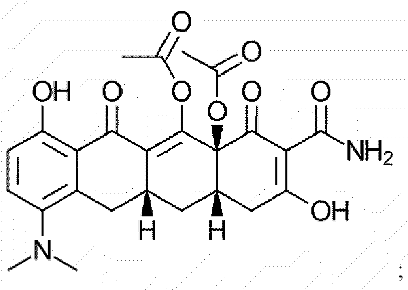 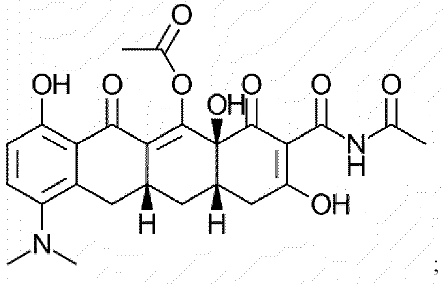 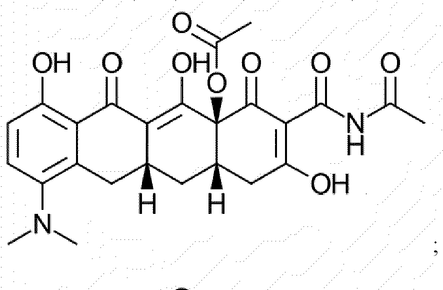 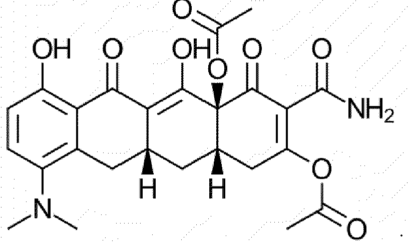 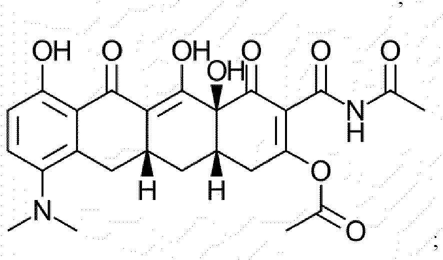 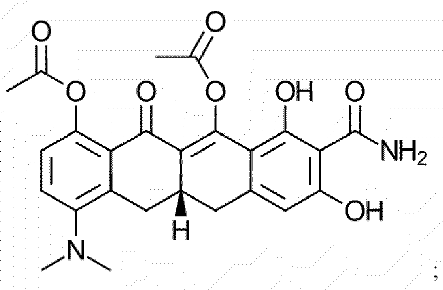 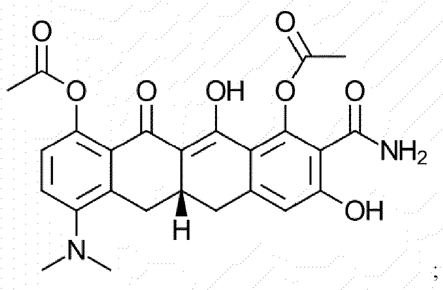 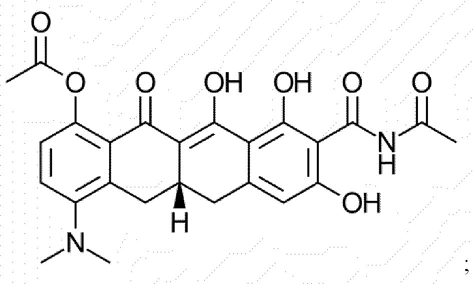 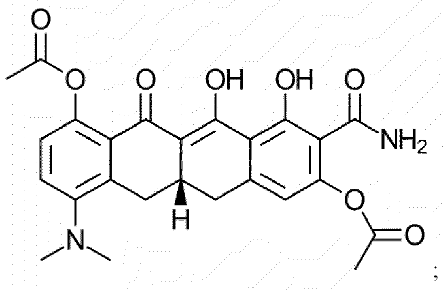 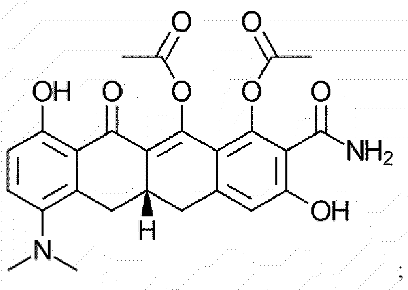 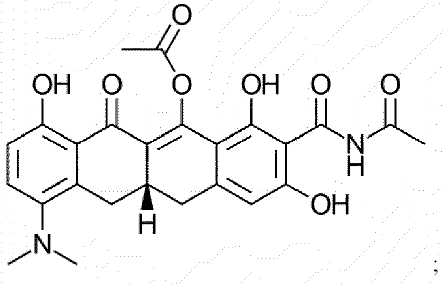 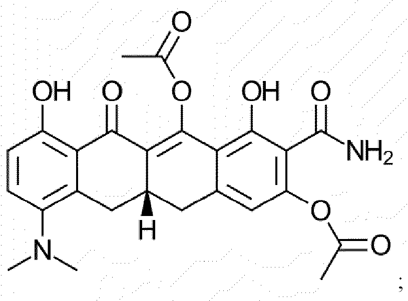 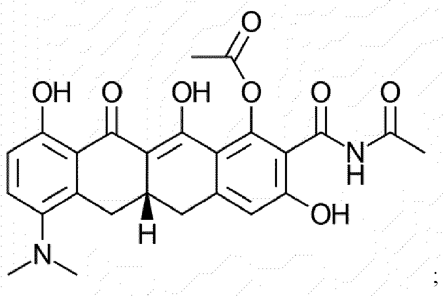 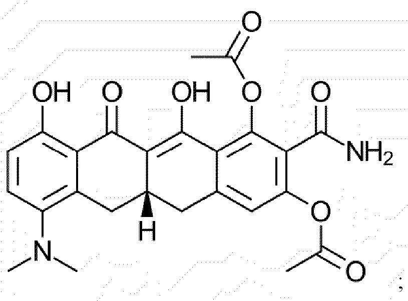 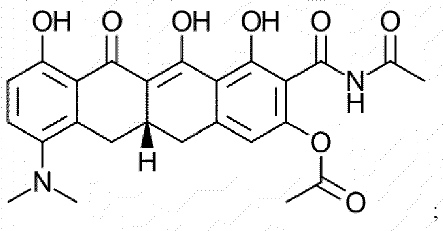 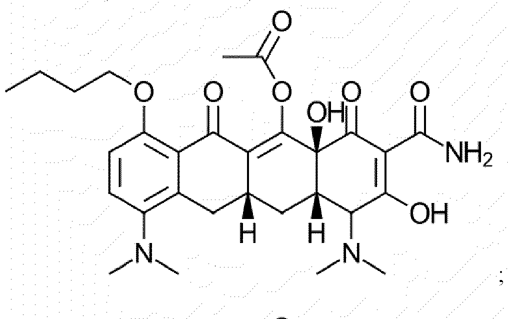 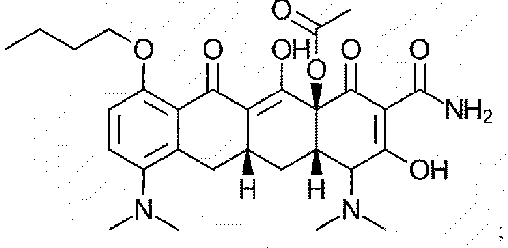 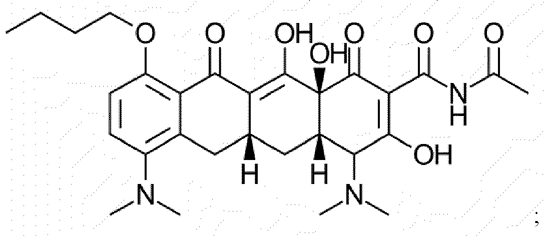 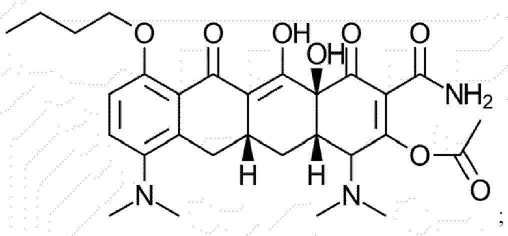 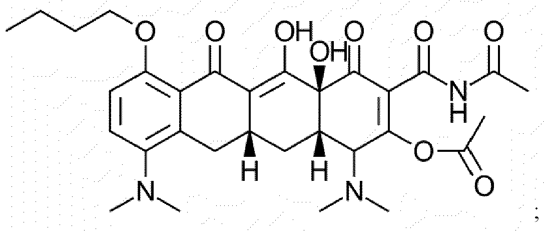 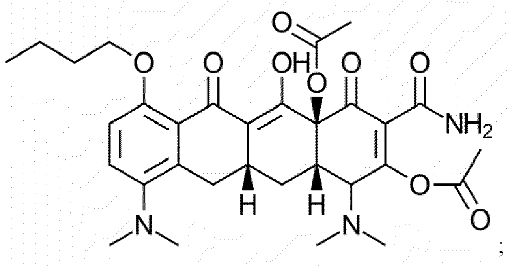 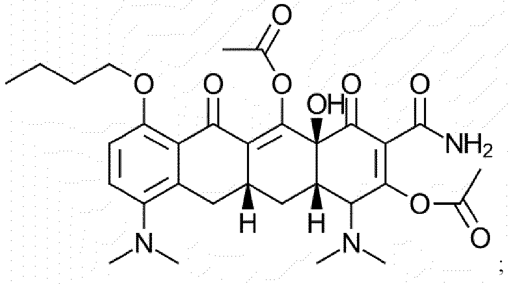 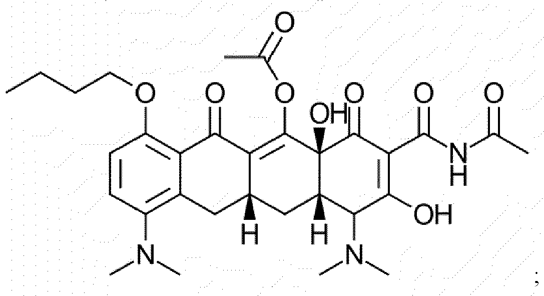 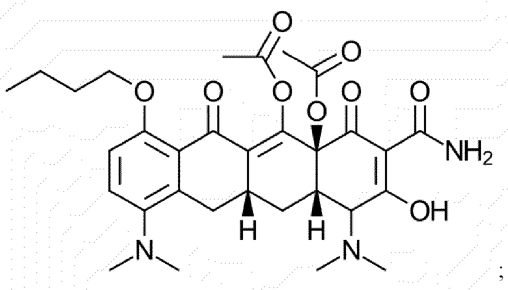 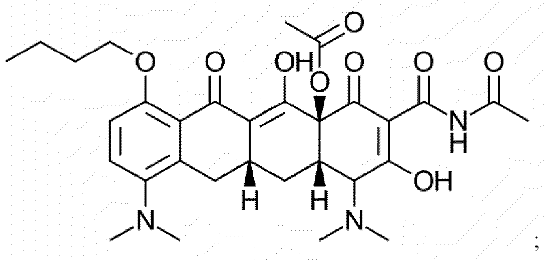 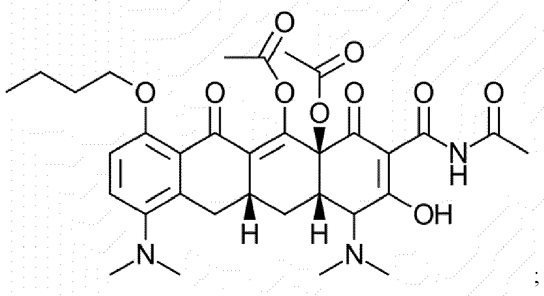 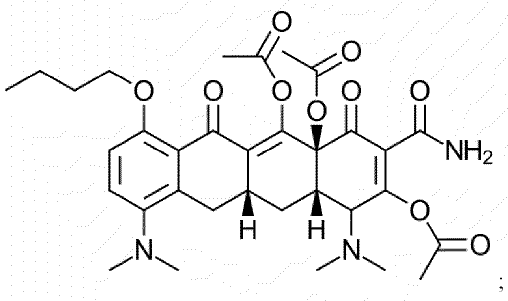 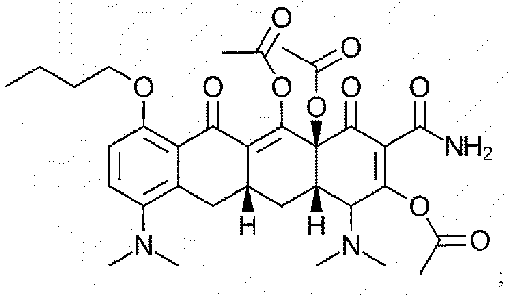 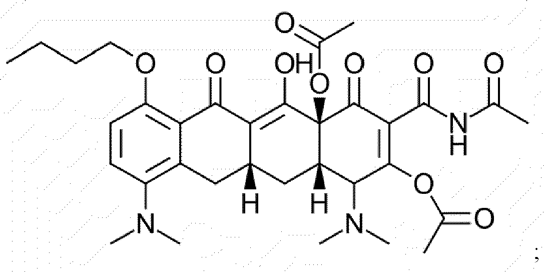 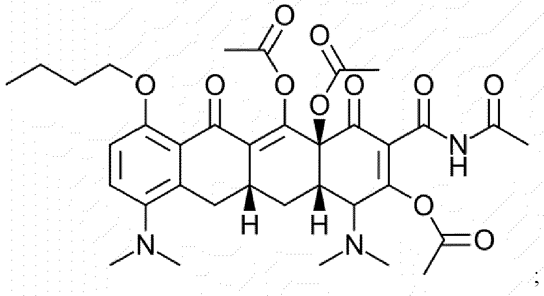 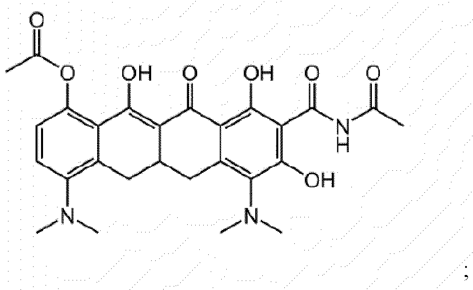 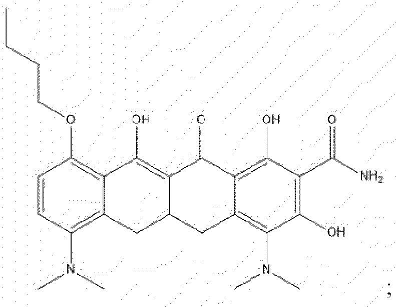 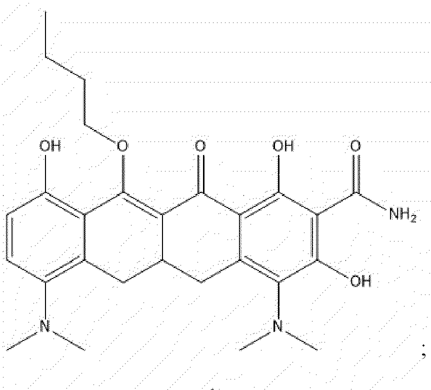 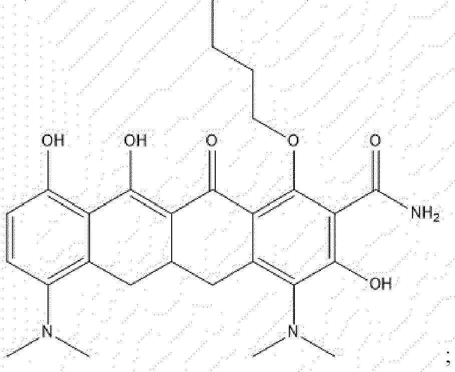 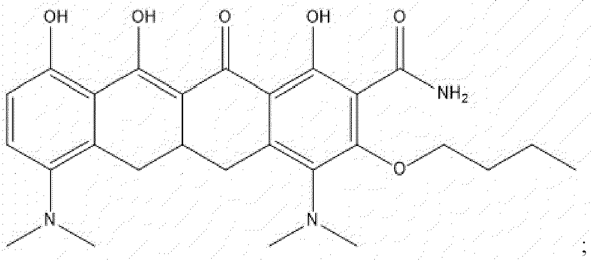 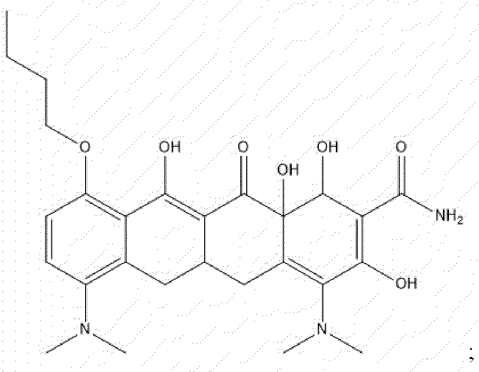 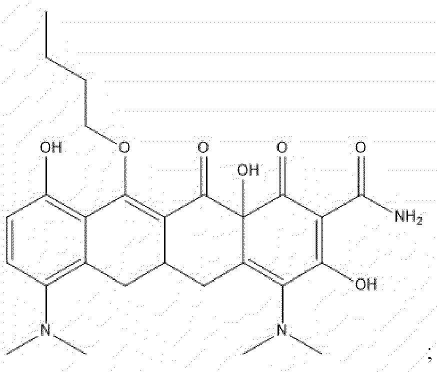 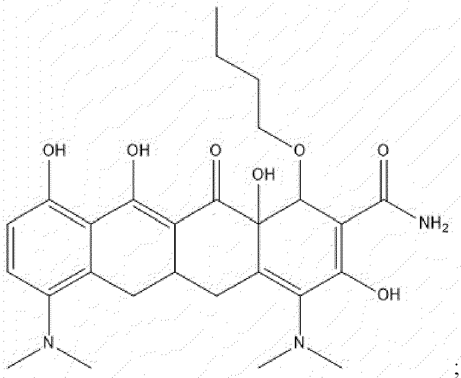 or

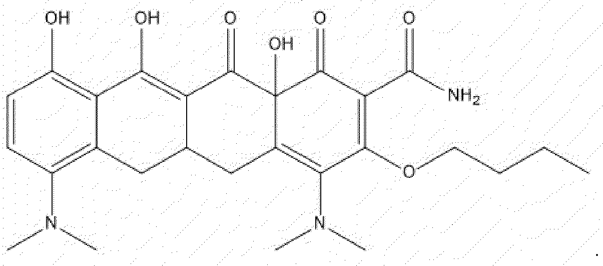

In one aspect, the molecule has reduced or substantially no antibacterial activity. In another aspect, the molecule has reduced or substantially no antifungal activity. Disclosed is providing the molecule in an amount that inhibits Alcohol Use Disorder (AUD), Substance Use Disorder (SUD), tobacco use, pain and inflammatory responses. The inflammatory response can be rheumatoid arthritis. The molecules may not inhibit a ribosome, e.g., a bacterial ribosome. The modification may at least one of: produces steric hindrance, blocks hydrogen bonding, or change coordination with divalent cations. In another aspect, the molecule is disposed in a pharmaceutically acceptable buffer, excipient, filler, or carrier. In another aspect, the molecule is formulated in a composition for administration orally, enterally, intramuscularly, parenterally, intravenously, or intraperitoneally.

In another embodiment, the present invention includes a pharmaceutical composition comprising a compound of Formula 1 to 20 or 25 to 48, pharmaceutically acceptable salts, and tautomers thereof.

In another embodiment, the present invention includes the tetracycline of Formula 1 to 20 or 25 to 48 for use in a method of treating Alcohol Use Disorder (AUD), Substance Use Disorder (SUD), tobacco use, pain, or a proinflammatory disorder comprising: optionally identifying a subject in need of treatment for Alcohol Use Disorder (AUD), Substance Use Disorder (SUD), tobacco use, pain, or a proinflammatory disorder and providing the subject with an effective amount of one or more modified tetracyclines of Formula 1 to 20 or 25 to 48 to ameliorate or eliminate the AUD, SUD, pain, or proinflammatory disorder. In one aspect, the molecule has reduced or substantially no antibacterial activity. In another aspect, the proinflammatory disorder is rheumatoid arthritis. In another aspect, the molecule has reduced or substantially no antifungal activity. In another aspect, the molecule inhibits Alcohol Use Disorder (AUD), Substance Use Disorder (SUD, pain and disorders involving potential inflammatory processes. Disclosed is a modified molecule that is a doxycycline, minocycline, or tigecycline or their tautomeric structures. The modification may at least one of: produces steric hindrance, blocks hydrogen bonding, or change coordination with divalent cations. In another aspect, the method further comprises providing the molecule in a pharmaceutically acceptable buffer, excipient, filler, or carrier. In another aspect, the method further comprises formulating a composition for administration orally, enterally, intramuscularly, parenterally, intravenously, or intraperitoneally.

Disclosed is a method of treating Alcohol Use Disorder (AUD), Substance Use Disorder (SUD), tobacco use, pain, or proinflammatory disorder comprising: identifying a subject in need of treatment for at least one of AUD, SUD, pain, or a proinflammatory disorder; and providing the subject with an effective amount of one or more modified tetracyclines of Formula 1 to 48 to ameliorate or eliminate the AUD, SUD, pain, or proinflammatory disorder and that reduced, or no, antimicrobial activity. The molecule may have reduced or substantially no antibacterial activity. The proinflammatory disorder may be rheumatoid arthritis. The molecule may have reduced or substantially no antifungal activity. The molecule may inhibit Alcohol Use Disorder (AUD), Substance Use Disorder (SUD, pain and disorders involving potential inflammatory processes. Tthe modified molecule may be a doxycycline, minocycline, or tigecycline or their tautomeric structures. The molecule may not inhibit a ribosome, e.g., a bacterial ribosome. The modification may at least one of: produces steric hindrance, blocks hydrogen bonding, or change coordination with divalent cations. The method may further comprise providing the molecule in a pharmaceutically acceptable buffer, excipient, filler, or carrier. The method may further comprises formulating a composition for administration orally, enterally, intramuscularly, parenterally, intravenously, or intraperitoneally.

Disclosed is a method of evaluating a candidate drug believed to be useful in treating Alcohol Use Disorder (AUD), Substance Use Disorder (SUD, including for opioids, tobacco use, pain, or proinflammatory disorder, the method comprising: a) measuring the Alcohol Use Disorder (AUD), Substance Use Disorder (SUD), tobacco use, pain, or proinflammatory disorder from a set of patients; b) administering a candidate drug to a first subset of the patients, and a placebo to a second subset of the patients, wherein the candidate drug is a modified doxycycline, minocycline, tigecycline and their tautomeric structures that have different combinations of halogen, acetyl ester, methyl ester, and diacetal having Formula 1 to 48; c) repeating step a) after the administration of the candidate drug or the placebo; and d) determining if the candidate drug reduces the Alcohol Use Disorder (AUD), Substance Use Disorder (SUD, pain, or proinflammatory disorders that is statistically significant as compared to any reduction occurring in the second subset of patients, wherein a statistically significant reduction indicates that the candidate drug is useful in treating Alcohol Use Disorder (AUD), Substance Use Disorder (SUD, tobacco use, pain, or proinflammatory disorders. The molecules may have moderate to no antibacterial activity. The molecules may have moderate to no antifungal activity. The molecule may be provided in an amount sufficient to inhibit Alcohol Use Disorder (AUD), Substance Use Disorder (SUD), pain and disorders involving potential inflammatory processes. The modified molecules may be a doxycycline, minocycline, or tigecycline or their tautomeric structures. The modification may at least one of: produces steric hindrance, blocks hydrogen bonding, or change coordination with divalent cations.

### BRIEF DESCRIPTION OF THE DRAWINGS

For a more complete understanding of the features and advantages of the present invention, reference is now made to the detailed description of the invention along with the accompanying figures and in which:
FIG. 1 shows a proposed mechanism by which ethanol consumption is reduced.
FIG. 2 shows the basic method for generating new compounds for treating Alcohol Use Disorder (AUD) that have low or no antimicrobial activity.
FIG. 3 shows that minocycline inhibits bacterial protein synthesis through binding to the 30S subunit of bacterial ribosomes.
FIGS. 4A to 4C show, FIG. 4A) Zone of inhibition assays show that CMMs lost antibacterial action against *E. coli* in 24 hour cell culture. FIG. 4B) Colony forming unit assays show that CMMs lost antibacterial action against E. *coli* in 24 hour cell culture. FIG. 4C) Colony forming unit assays show that CMMs lost antifungal action against C. *albicans* in 48 hour cell culture. MINO = minocycline, BeMAc = Butyl ether monoacetate minocycline, DeDiAc = De amino di acetyl minocycline, TRI = Tri acetyl minocycline.
FIGS. 5A to 5C show, FIG. 5A) CMMs reduced acute ethanol consumption in the Drinking in the Dark model of AUD. MINO, DeDiAc, and TRI reduced ethanol consumption by over 50% by intraperitoneal injection (Left). MINO, DeDiAc, TRI, and BeMAc reduced ethanol consumption to a lesser degree by gavage (Right). FIG. 5B) Dose response curve for DeDIAc in female mice by gavage. 5C) Dose response curve for DeDIAc in male mice by gavage. MINO = minocycline, BeMAc = Butyl ether monoacetate minocycline, DeDiAc = De amino di acetyl minocycline, TRI = Tri acetyl minocycline. *p < .05 compared to controls.
FIG. 6 shows the method of screening modifications to minocycline, CMM analogs were confirmed for purity structure utilizing HPLC, NMR and LC-MS. ZOI and CFU assays were to confirm loss of antibiotic activity, and alcohol consumption was tested in C67BL/6J mice.
FIG. 7 shows the site of chemical modification on minocycline, and for reference, the solved structure of ribosome with minocycline. Schedlbauer et al., Antimicrobial Agents and Chemotherapy. 59(5): 2849-2854, 2015.
FIG. 8A to 8D shows the effect of Butyl ether minocycline lost antibiotic activity compared to minocycline.
FIG. 9 shows a flowchart for the mechanism of action of minocycline related to autophagy, inflammation, and angiogenesis.
FIG. 10 shows the cellular signaling pathways for COX2, BNIP3, acidic vacuoles, and GFP-LC3 puncta.
FIGS. 11A to 11C show Deamino diacetyl minocycline mass, purity, and structure were confirmed using LC-MS (FIG. 11B), and HPLC (FIG. 11C), which was further confirmed with NMR (not shown).
FIGS. 12A to 12D show the zone of inhibition and colony forming units assays showed loss of antibiotic activity for deamino diacetyl minocycline FIG. 12A and 12C, respectively, compared to minocycline FIGS. 12B and 12D, respectively.
FIG. 13 shows the basic experimental set-up for the rheumatoid arthritis. The RA model was induced with 5-monoclonal antibodies directed against collagen type II followed by an E. coli lipopolysaccharide adjuvant (CIA, Chondrex, Redmond, WA) in female and male DBA/1J mice. Groups were treated with saline or 75mg/kg of CMM i.p. RA was quantified using caliper measurements, infrared heat signatures, arthritis scoring, hotplate (42 ⁰C) and Von Frey filament assays.
FIG. 14 shows CMM reduced paw swelling by approximately 50% as measured by caliper (mm). Date is shown as increase over baseline measurements.
FIG. 15 shows the CMM reduced pressure nociception in RA mice as measured by the von Frey filament assay. Induced mice that received only a saline treatment saw a roughly 40% increase in sensitivity after 21 days as compared to their baseline. This sensitivity was virtually eliminated from the group that received the MMC injections.
FIGS. 16A and 16B show: FIG. 16A are photos resulting from the injection with the Chondrex antibody cocktail lead to severe inflammation of all the phalangeal, metatarsal, and ankle joints along with a loss of anatomical definition in the front paw. MCC significantly reduced the RA severity with an approximately 70% reduction in arthritis scoring versus the induced group shown in FIG. 16B. Improvement was clear in both female and male mice.

### DETAILED DESCRIPTION OF THE INVENTION

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not limit the invention, except as outlined in the claims.

The present invention is directed to modification of tetracyclines with R6'=H, such as doxycycline, minocycline and tigecycline, by acetylation or other modification such that the molecule loses most, or all, of its antibacterial properties, yet retain the ability to reduce negative traits related to alcohol consumption, including, but not limited to high drinking levels, withdrawal symptoms and increased sensitization and duration of pain, and reduce tobacco consumption. In addition, the novel molecules can also be used to treat disorders that have an innate immune component. The invention involves any tetracycline that does not bind to its site of action in a bacterium. The modification of tetracyclines at R6' in which R6' is not H, may act by different mechanisms, such as, steric hindrance, stereo chemistry, lack of hydrogen binding, transport into the bacteria, etc., however, the modification must allow it to still retain its ability to reduce high drinking levels, withdrawal symptoms and increased sensitization and duration of pain, and reduce tobacco consumption.

Only three pharmacotherapeutic treatments for Alcohol Use Disorder (AUD) are FDA approved and none are widely used (<10% of AUD patients) or show a strong effect to reduce risky- or dependence-based drinking in the long-term (<20% see sustained decreased drinking outcomes). Unfortunately, approximately 10% of the US population suffers from AUD and over 5% of all medical morbidities share risky ethanol consumption as an underlying issue. As a consequence, intoxication, in general, and 'alcohol addiction' (severe AUD), in particular, are important clinical problems. Given the limited pharmacotherapeutic choice, there is a compelling need for continued development of new treatments across the AUD spectrum (mild to severe DSM-V classification). In fact, improved treatments targeting high alcohol consumption and withdrawal-related symptoms are desirable as precipitating withdrawal can be a medical emergency with risk for death. To date, drugs targeting drinking do not protect against withdrawal, and drugs used to reduce withdrawal symptoms are often co-addictive with alcohol.

The present inventors recently showed that tetracycline analogs were preclinically efficacious to reduce high alcohol consumption, withdrawal symptoms and alcohol-mediated pain sensitization and now have exciting preliminary data showing efficacy for an improved chemically modified minocycline (CMM) (Bergeson, Blanton, et al. 2016; Martinez et al. 2016; Bergeson, Nipper, et al. 2016; Syapin, Martinez, Curtis, Marquardt, Allison, Groot, Baby, Al-Hasan, Segura, et al. 2016). Further, the inventors have previously found tetracycline analogs, including doxycycline, minocycline, and tigecycline to be efficacious against various aspects of Alcohol Use Disorder (AUD), including cessation of drinking, withdrawal symptoms and sensitization and increased duration of pain (Bergeson, Blanton, et al. 2016; Martinez et al. 2016; Bergeson, Nipper, et al. 2016; Syapin, Martinez, Curtis, Marquardt, Allison, Groot, Baby, Al-Hasan, Segura-Ulate, et al. 2016; Agrawal et al. 2014; Agrawal et al. 2011).

However, despite these encouraging results, the present inventors have found that the effect of these tetracyclines was through a central nervous system (CNS) function and not mediated in any part by changes in resident bacteria. The data shown is key to understanding that the tetracyclines could be modified to remove the antibiotic property and still remain useful for AUD treatment. Other literature suggests that alcohol effects may be mediated, at least in part, by bacteria or their components (Blednov et al. 2011); for a recent review, see (Montesinos, Alfonso-Loeches, and Guerri 2016). However, despite this controversy in the literature, the present invention is the first to show that the action of the known CMMs does not require antibiotic properties.

Thus, the present inventors recognized for the first time that the mechanism of action of the CMM for use in Alcohol Use Disorder, pain and other disorders involving potential inflammatory processes does not involve tetracycline's general antibiotic properties. Thus, the present inventors tested several tetracyclines to determine structural or functional components that contributed to the AUD treatment efficacy. As shown in Table 1, it appears that the C6' hydrogen is, at least in part necessary to convey the positive action on AUD-related traits, but not those known to bind to the A-site of the bacterial ribosome (Schedlbauer et al. 2015).

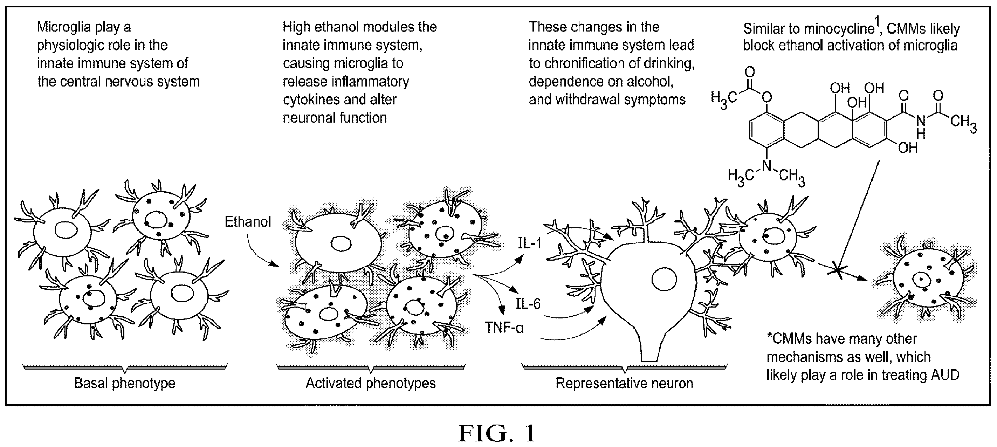

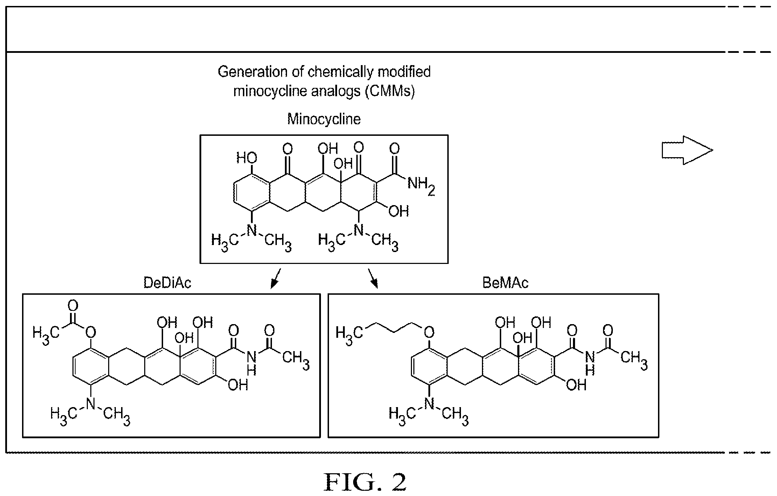

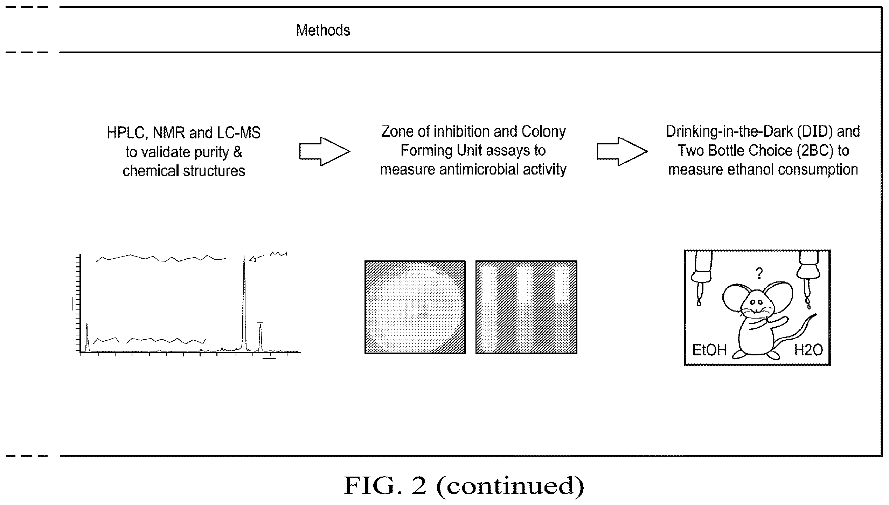

Table 1. Of seven tetracyclines tested against AUD traits (Syapin, Martinez, Curtis, Marquardt, Allison, Groot, Baby, Al-Hasan, Segura-Ulate, et al. 2016) only doxycycline, minocycline and tigecycline were effective. Shown in grey highlight is that the R6' group is the only difference between the effective and non-effective tetracycline drugs, and together with our unpublished data in FIGS. 1 and 2, indicated that the structure of the molecule could be modified to lose its bacterial ribosome binding component. Removal of anti-microbial properties should reduce side effects and avoid increased drug resistance.

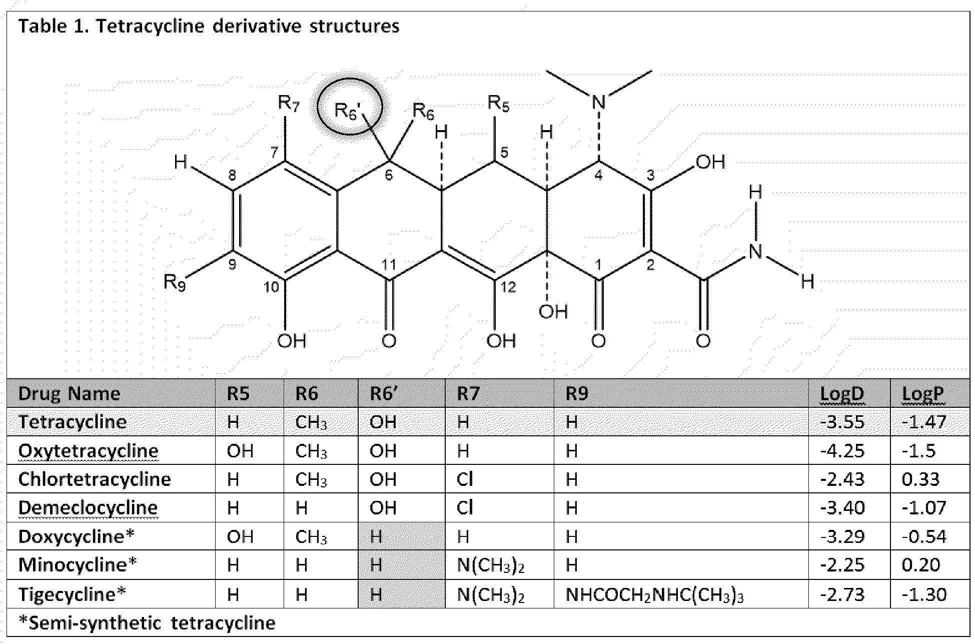

The compounds of the present invention have the following formulas, and include pharmaceutically acceptable salts, pro-drugs, biologically active metabolites, and tautomers thereof are listed in Table 2.

**Table 2 includes a list that compares additional minocycline derivatives of the present invention to minocycline and, e.g., its HCl salt.**

| Name | IUPAC Name | Structure |
|---|---|---|
| | (5aR,6aS,10aS)-11-Acetoxy-9-carbamoyl-4-(dimethylamino)-8,10a-dihydroxy-10,12-dioxo-5,5a,6,6a,7,10a-hexahydro-1-naphthacenyl acetate) | 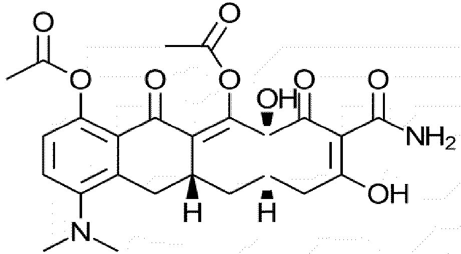 |
| | Formula 1 | |
| | (5aR,6aS,10aS)-10a-Acetoxy-9-carbamoyl-4-(dimethylamino)-8,11-dihydroxy-10,12-dioxo-5,5a,6,6a,7,10a-hexahydro-1-naphthacenyl acetate) | 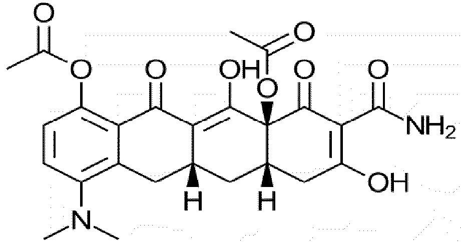 |
| | Formula 2 | |
| Deamino Diacetyl Minocycline | (5aR,6aS,10aS)-9-(N-Acetylcarbamoyl)-4-(dimethylamino)-8,10a,11-trihydroxy-10,12-dioxo-5,5a,6,6a,7,10a-hexahydro-1-naphthacenyl acetate) | 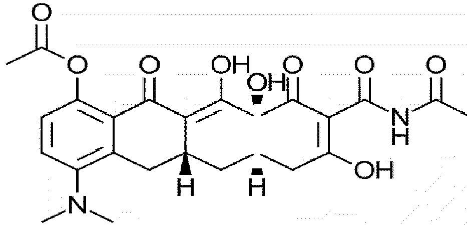 |
| | Formula 3 | |
| | (4aS,11aR,12aS)-7-Acetoxy-3-carbamoyl-10-(dimethylamino)-4a,5-dihydroxy-4,6-dioxo-1,4a,11,11a,12,12a-hexahydro-2-naphthacenyl acetate) | 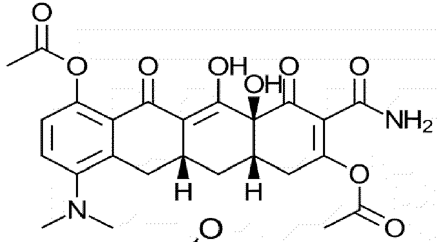 |
| | Formula 4 | |
| | (4aS,11aR,12aS)-2-Acetoxy-3-carbamoyl-10-(dimethylamino)-4a,7-dihydroxy-4,6-dioxo-1, 4a,11,11a,12,12a-hexahydro-5-naphthacenyl acetate) | 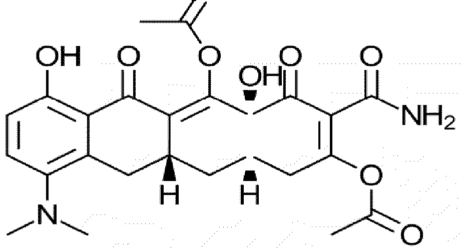 |
| | Formula 5 | |
| | (4aS,11aR,12aS)-4a-Acetoxy-3-carbamoyl-10-(dimethylamino)-2,7-dihydroxy-4,6-dioxo-1,4a,11,11a,12,12a-hexahydro-5-naphthacenyl acetate) | 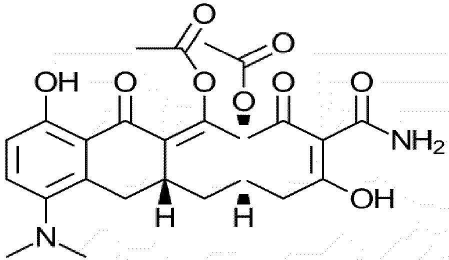 |
| | Formula 6 | |
| | (4aS,11aR,12aS)-3-(N-Acetylcarbamoyl)-10-(dimethylamino)-2,4a,7-trihydroxy-4,6-dioxo-1,4a,11,11a,12,12a-hexahydro-5-naphthacenyl acetate) | 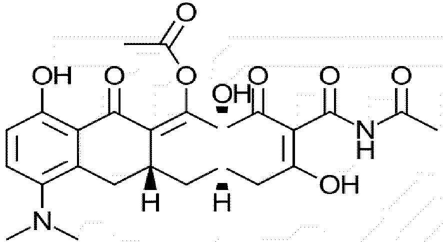 |
| | Formula 7 | |
| | (4aS,11aR,12aS)-3-(N-Acetylcarbamoyl)-10-(dimethylamino)-2,5,7-trihydroxy-4,6-dioxo-1,4a,11,11a,12,12a-hexahydro-4a-naphthacenyl acetate) | 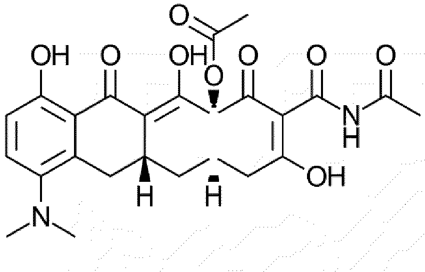 |
| | Formula 8 | |
| | (4aS,11aR,12aS)-2-Acetoxy-3-carbamoyl-10-(dimethylamino)-5,7-dihydroxy-4,6-dioxo-1,4a,11,11a,12,12a-hexahydro-4a-naphthacenyl acetate) | 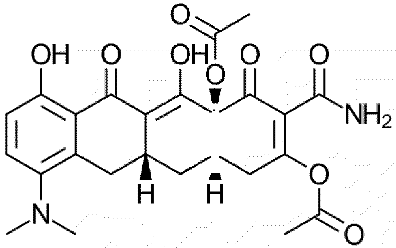 |
| | Formula 9 | |
| | (4aS,11aR,12aS)-3-(N-Acetylcarbamoyl)-10-(dimethylamino)-4a,5,7-trihydroxy-4,6-dioxo-1,4a,11,11a,12,12a-hexahydro-2-naphthacenyl acetate) | 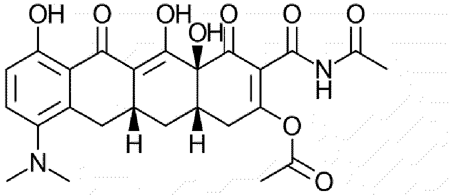 |
| | Formula 10 | |
| | (*R*)-11-Acetoxy-9-carbamoyl-4-(dimethylamino)-8,10-dihydroxy-12-oxo-5a,6-dihydro-5*H*-naphthacen-1-yl acetate | 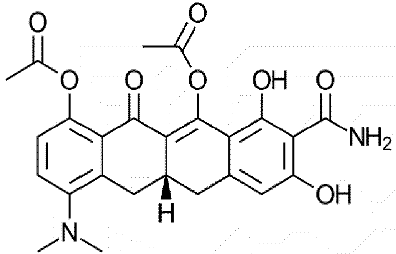 |
| | Formula 11 | |
| | (*R*)-10-Acetoxy-2-carbamoyl-7-(dimethylamino)-3,12-dihydroxy-11-oxo-5a,6-dihydro-5*H*-naphthacen-1-yl acetate | 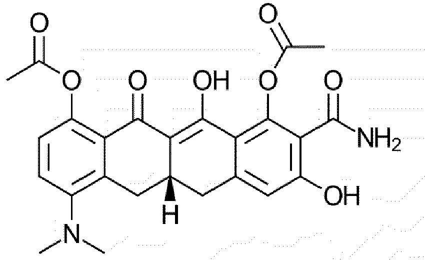 |
| | Formula 12 | |
| | (*R*)-9-(*N*-Acetylcarbamoyl)-4-(dimethylamino)-8,10,11-trihydroxy-12-oxo-5a,6-dihydro-5*H*-naphthacen-1-yl acetate | 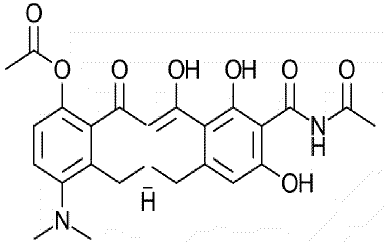 |
| | Formula 13 | |
| | (*R*)-8-Acetoxy-9-carbamoyl-4-(dimethylamino)-10,11-dihydroxy-12-oxo-5a,6-dihydro-5*H*-naphthacen-1-yl acetate | 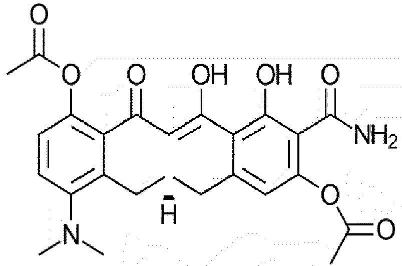 |
| | Formula 14 | |
| | (*R*)-12-Acetoxy-2-carbamoyl-7-(dimethylamino)-3,10-dihydroxy-11-oxo-5a,6-dihydro-5*H*-naphthacen-1-yl acetate | 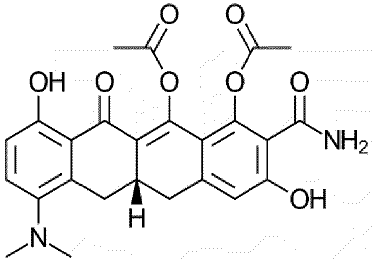 |
| | Formula 15 | |
| | ((R)-3-(N-Acetylcarbamoyl)-10-(dimethylamino)-2,4,7-trihydroxy-6-oxo-11a,12-dihydro-11H-naphthacen-5-yl acetate) | 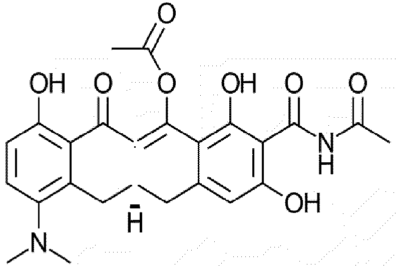 |
| | Formula 16 | |
| | (*R*)-5-Acetoxy-3-carbamoyl-10-(dimethylamino)-4,7-dihydroxy-6-oxo-11a,12-dihydro-11*H*-naphthacen-2-yl acetate | 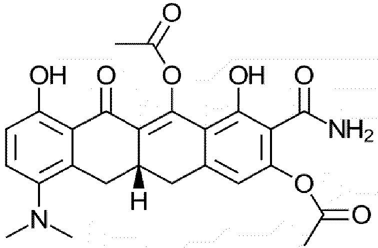 |
| | Formula 17 | |
| | (R)-2-(N-Acetylcarbamoyl)-7-(dimethylamino)-3,10,12-trihydroxy-11-oxo-5a,6-dihydro-5H-naphthacen-1-yl acetate | 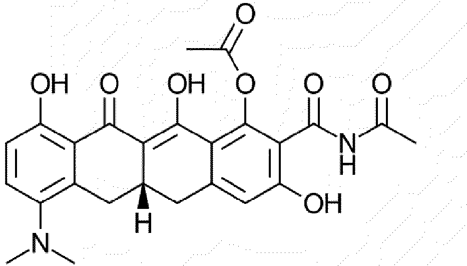 |
| | Formula 18 | |
| | (*R*)-3-Acetoxy-2-carbamoyl-7-(dimethylamino)-10,12-dihydroxy-11-oxo-5a,6-dihydro-5*H*-naphthacen-1-yl acetate | 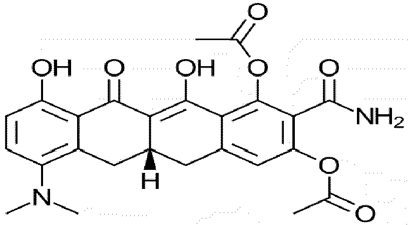 |
| | Formula 19 | |
| | (*R*)-3-(*N*-Acetylcarbamoyl)-10-(dimethylamino)-4,5,7-trihydroxy-6-oxo-11a,12-dihydro-11*H*-naphthacen-2-yl acetate | 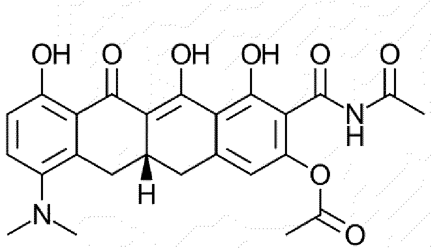 |
| | Formula 20 | |
| Methyl Ether Minocycline | (4aS,5aR,12aS)-4,7-Bis(dimethylamino)-3,12,12a-trihydroxy-10-methoxy-1,11-dioxo-4,4a,5,5a,6,12a-hexahydro-2-naphthacenecarboxamide) | 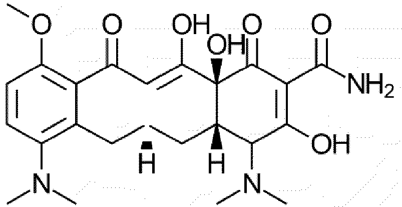 |
| | Comparative Formula 21 | |
| Ethyl Ether Minocycline | (4aS,5aR,12aS)-4,7-Bis(dimethylamino)-10-ethoxy-3,12,12a-trihydroxy-1,11-dioxo-4,4a,5,5a,6,12a-hexahydro-2-naphthacenecarboxamide) | 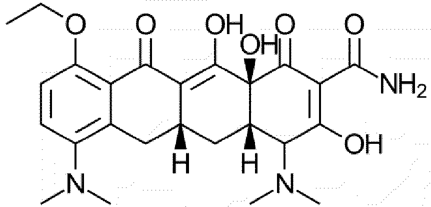 |
| | Comparative Formula 22 | |
| Propyl Ether Minocycline | (4aS,5aR,12aS)-4,7-Bis(dimethylamino)-3,12,12a-trihydroxy-1,11-dioxo-10-propoxy-4,4a,5,5a,6,12a-hexahydro-2-naphthacenecarboxamide) | 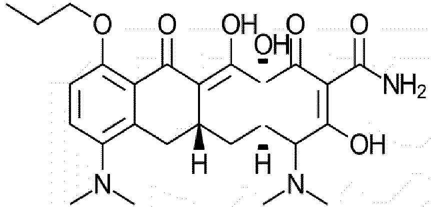 |
| | Comparative Formula 23 | |
| Butyl Ether Minocycline | (4aS,5aR,12aS)-10-Butoxy-4,7-bis(dimethylamino)-3,12,12a-trihydroxy-1,11-dioxo-4,4a,5,5a,6,12a-hexahydro-2-naphthacenecarboxamide) | 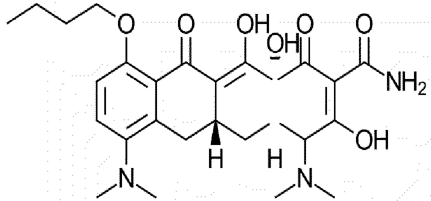 |
| | Comparative Formula 24 | |
| Butyl Ether Monoacetyl Minocycline | (4aS,11aR,12aS)-7-Butoxy-3-carbamoyl-1,10-bis(dimethylamino)-2,4a-dihydroxy-4,6-dioxo-1,4a,11,11a,12,12a-hexahydro-5-naphthacenyl acetate) | 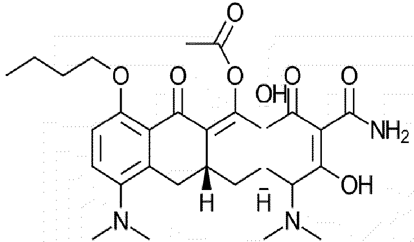 |
| | Formula 25 | |
| | (4aS,11aR,12aS)-7-Butoxy-3-carbamoyl-1,10-bis(dimethylamino)-2,5-dihydroxy-4,6-dioxo-1,4a,11,11a,12,12a-hexahydro-4a-naphthacenyl acetate) | 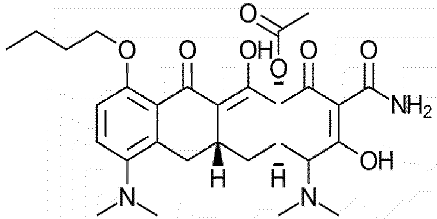 |
| | Formula 26 | |
| | (N-Acetyl-(4aS,5aR,12aS)-10-butoxy-4,7-bis(dimethylamino)-3,12,12a-trihydroxy-1,11-dioxo-4,4a,5,5a,6,12a-hexahydro-2-naphthacenecarboxamide) | 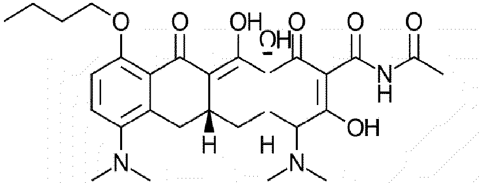 |
| | Formula 27 | |
| | (4aS,11aR,12aS)-7-Butoxy-3-carbamoyl-1,10-bis(dimethylamino)-4a,5-dihydroxy-4,6-dioxo-1,4a,11,11a,12,12a-hexahydro-2-naphthacenyl acetate) | 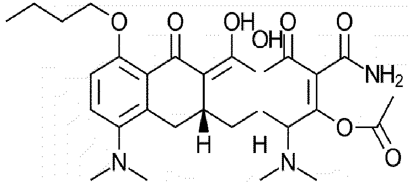 |
| | Formula 28 | |
| Butyl Ether Diacetyl | (4aS,11aR,12aS)-3-(N-Acetylcarbamoyl)-7-butoxy-1,10-bis(dimethylamino)-4a,5-dihydroxy-4,6-dioxo-1,4a,11,11a,12,12a-hexahydro-2-naphthacenyl acetate) | 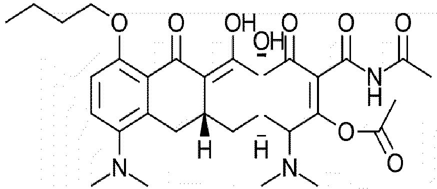 |
| | Formula 29 | |
| | (4aS,11aR,12aS)-2-Acetoxy-7-butoxy-3-carbamoyl-1,10-bis(dimethylamino)-5-hydroxy-4,6-dioxo-1,4a,11,11a,12,12a-hexahydro-4a-naphthacenyl acetate) | 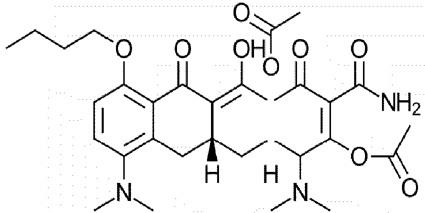 |
| | Formula 30 | |
| | (4aS,11aR,12aS)-2-Acetoxy-7-butoxy-3-carbamoyl-1,10-bis(dimethylamino)-4a-hydroxy-4,6-dioxo-1,4a,11,11a,12,12a-hexahydro-5-naphthacenyl acetate) | 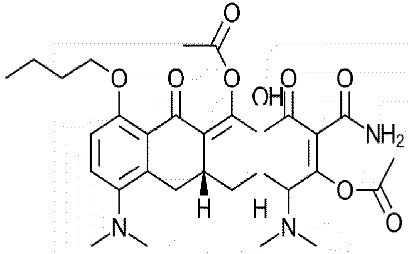 |
| | Formula 31 | |
| | (4aS,11aR,12aS)-3-(N-Acetylcarbamoyl)-7-butoxy-1,10-bis(dimethylamino)-2,4a-dihydroxy-4,6-dioxo-1,4a,11,11a,12,12a-hexahydro-5-naphthacenyl acetate) | 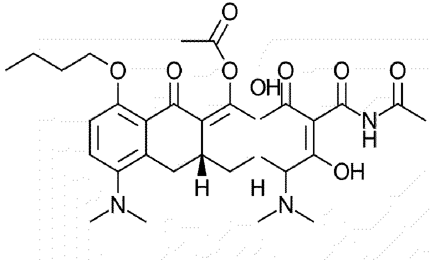 |
| | Formula 32 | |
| | (4aS,11aR,12aS)-4a-Acetoxy-7-butoxy-3-carbamoyl-1,10-bis(dimethylamino)-2-hydroxy-4,6-dioxo-1,4a,11,11a,12,12a-hexahydro-5-naphthacenyl acetate) | 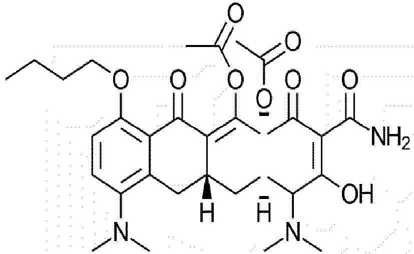 |
| | Formula 33 | |
| | (4aS,11aR,12aS)-3-(N-Acetylcarbamoyl)-7-butoxy-1,10-bis(dimethylamino)-2,5-dihydroxy-4,6-dioxo-1,4a,11,11a,12,12a-hexahydro-4a-naphthacenyl acetate) | 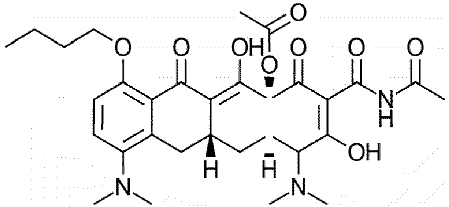 |
| | Formula 34 | |
| Buty Ether Triacetyl Minocycline | (4aS,11aR,12aS)-3-(N-Acetylcarbamoyl)-4a-acetoxy-7-butoxy-1,10-bis(dimethylamino)-2-hydroxy-4,6-dioxo-1,4a,11,11a,12,12a-hexahydro-5-naphthacenyl acetate) | 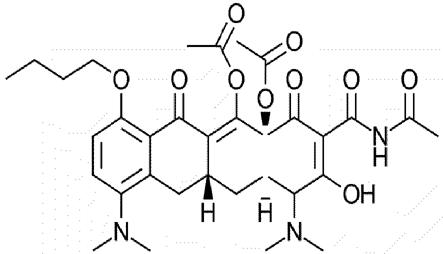 |
| | Formula 35 | |
| | (4aS,11a R,12aS)-2,4a-Diacetoxy-7-butoxy-3-carbamoyl-1,10-bis(dimethylamino)-4,6-dioxo-1,4a,11,11a,12,12a-hexahydro-5-naphthacenyl acetate) | 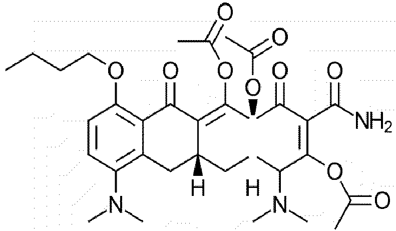 |
| | Formula 36 | |
| | (4aS,11aR,12aS)-3-(N-Acetylcarbamoyl)-2-acetoxy-7-butoxy-1,10-bis(dimethylamino)-4a-hydroxy-4,6-dioxo-1,4a,11,11a,12,12a-hexahydro-5-naphthacenyl acetate) | 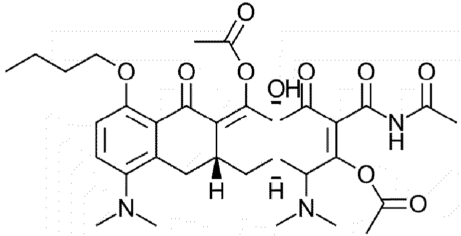 |
| | Formula 37 | |
| | (4aS,11aR,12aS)-3-(N-Acetylcarbamoyl)-2-acetoxy-7-butoxy-1,10-bis(dimethylamino)-5-hydroxy-4,6-dioxo-1,4a,11,11a,12,12a-hexahydro-4a-naphthacenyl acetate) | 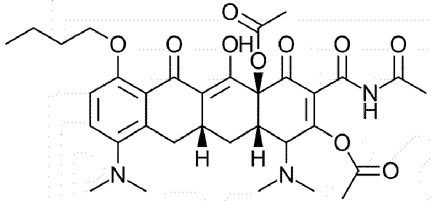 |
| | Formula 38 | |
| Butyl Ether Tetra Acetyl Minocycline | (4aS,11aR,12aS)-3-(N-Acetylcarbamoyl)-2,4a-diacetoxy-7-butoxy-1,10-bis(dimethylamino)-4,6-dioxo-1,4a,11,11a,12,12a-hexahydro-5-naphthacenyl acetate | 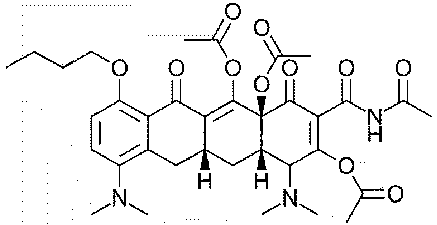 |
| | Formula 39 | |

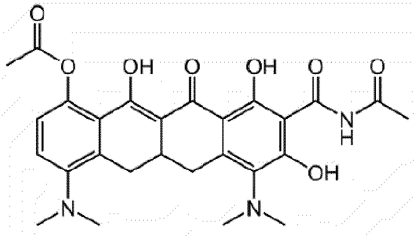

Di acetyl minocycline, Formula 40; 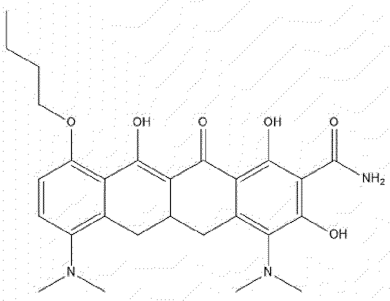 Butyl ether minocycline 1, Formula 41; 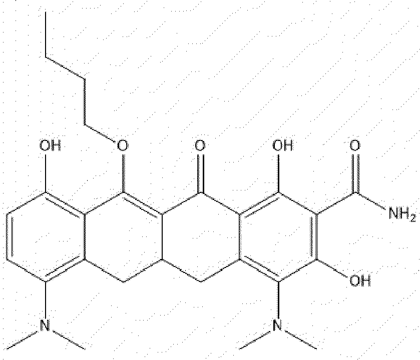 Butyl ether minocycline 2, Formula 42; 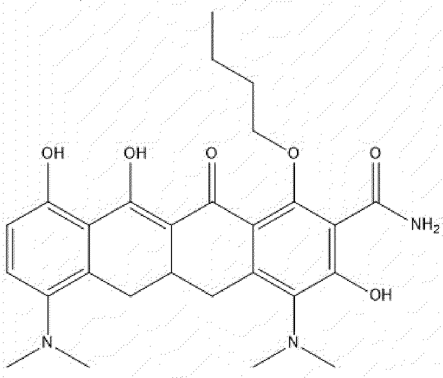 Butyl ether minocycline 3, Formula 43; 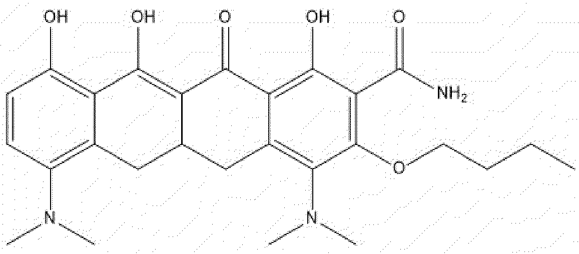 Butyl ether minocycline 4, Formula 44; 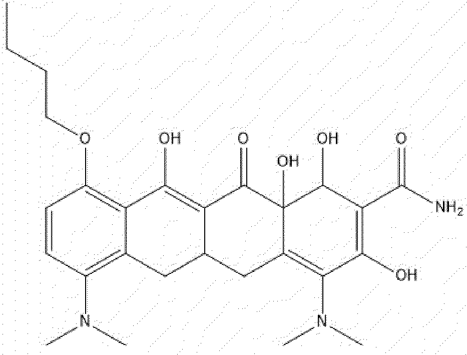 Butyl ether minocycline 5, Formula 45; 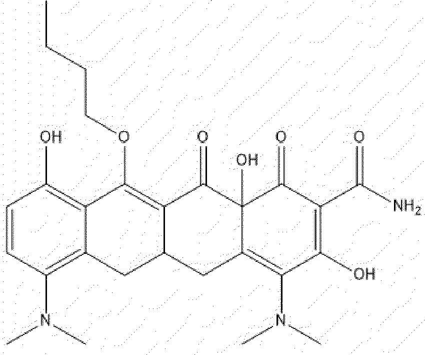 Butyl ether minocycline 7, Formula 46; 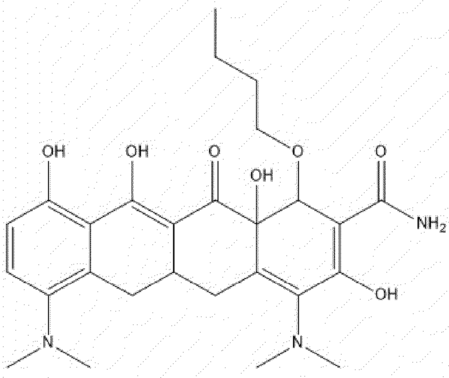 Butyl ether minocycline 6, Formula 47; 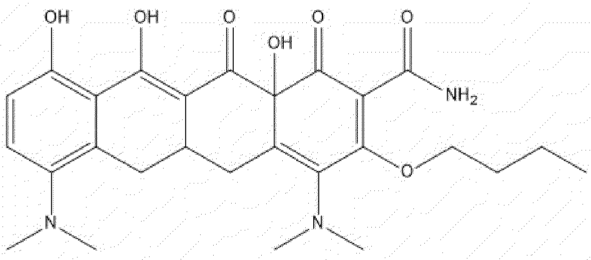 Butyl ether minocycline 8, Formula 48.

Example 1. Reduction of Ethanol Consumption by Chemically Modified Minocycline Compounds.

Alcohol Use Disorder (AUD) is the third leading cause of preventable morbidity in the USA, but < 20% of patients who receive pharmacologic intervention with currently approved medications achieve remission. Minocycline, an antimicrobial, has also shown promise as a treatment for AUD due to its non-antimicrobial mechanisms of action. However, the antimicrobial properties of minocycline causes problems when using it for non-infectious diseases. The inventors modified minocycline to remove the antimicrobial properties, but keep the AUD treating properties.

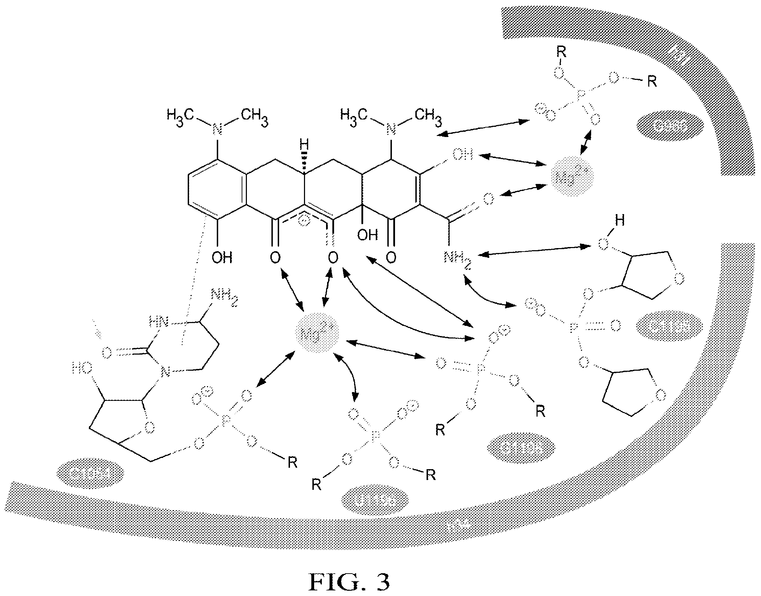

FIG. 1 shows a proposed mechanism by which ethanol consumption is reduced. FIG. 2 shows the basic method for generating new compounds for treating Alcohol Use Disorder (AUD) that have low or no antimicrobial activity. FIG. 3 shows that minocycline inhibits bacterial protein synthesis through binding to the 30S subunit of bacterial ribosomes.

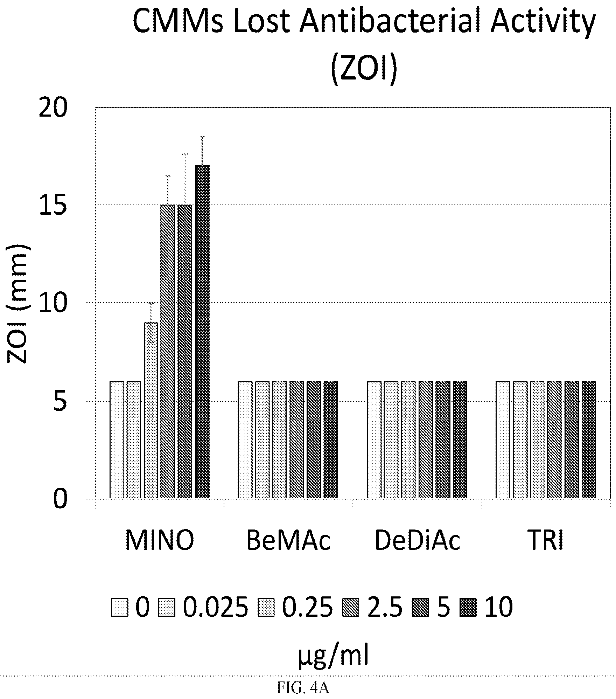

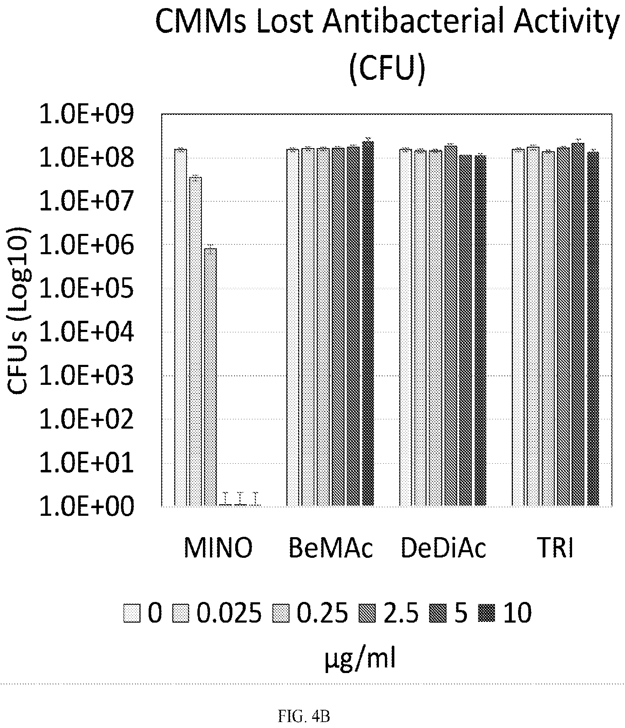

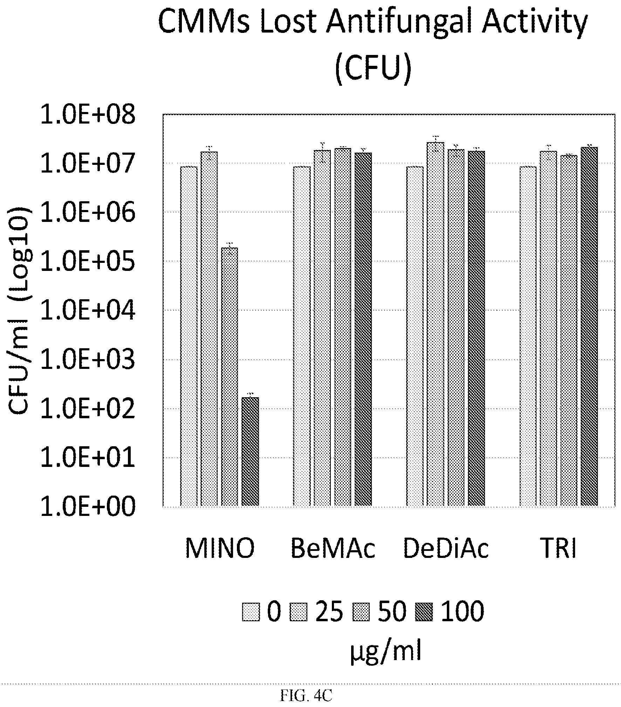

FIGS. 4A to 4C show, 4A) Zone of inhibition assays show that CMMs lost antibacterial action against *E. coli* in 24 hour cell culture. 4B) Colony forming unit assays show that CMMs lost antibacterial action against E. *coli* in 24 hour cell culture. 4C) Colony forming unit assays show that CMMs lost antifungal action against C. *albicans* in 48 hour cell culture. MINO = minocycline, BeMAc = Butyl ether monoacetate minocycline, DeDiAc = De amino di acetyl minocycline, TRI = Tri acetyl minocycline.

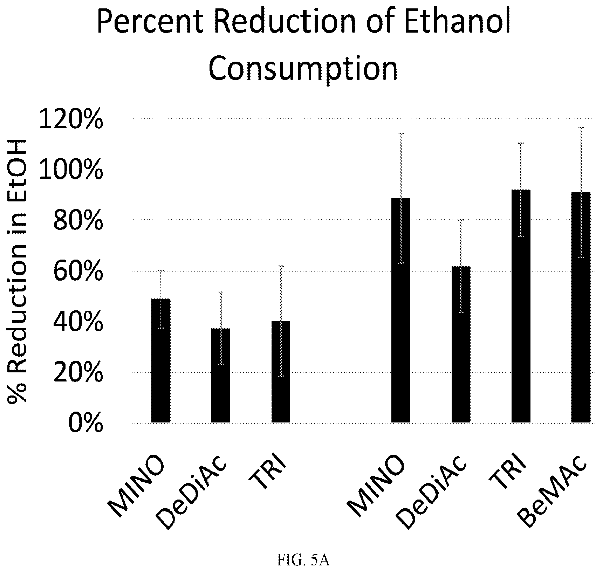

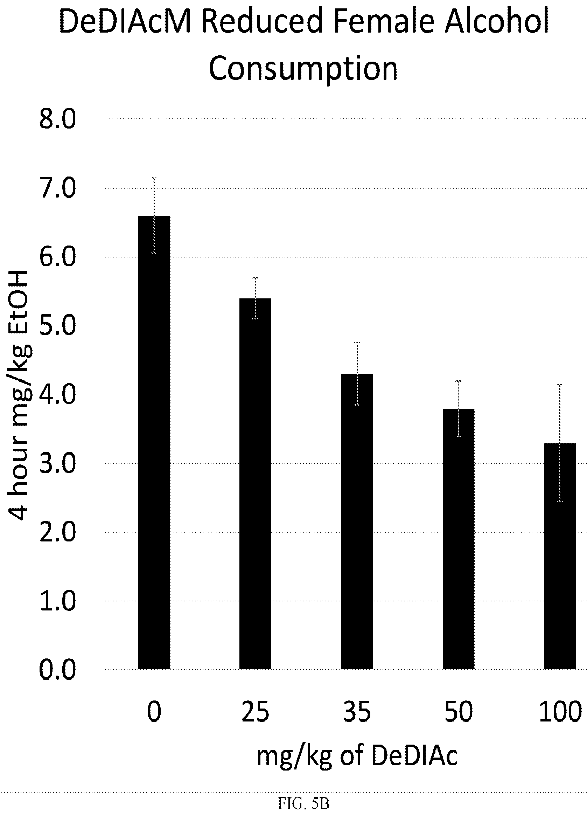

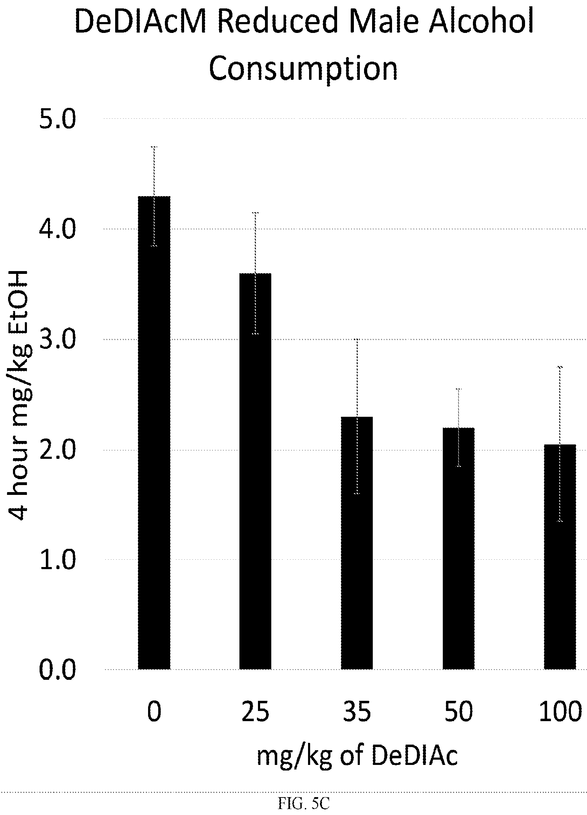

FIGS. 5A to 5C show, 5A) CMMs reduced acute ethanol consumption in the Drinking in the Dark model of AUD. MINO, DeDiAc, and TRI reduced ethanol consumption by over 50% by intraperitoneal injection (Left). MINO, DeDiAc, TRI, and BeMAc reduced ethanol consumption to a lesser degree by gavage (Right). 5B) Dose response curve for DeDIAc in female mice by gavage. 5C) Dose response curve for DeDIAc in male mice by gavage. MINO = minocycline, BeMAc = Butyl ether monoacetate minocycline, DeDiAc = De amino di acetyl minocycline, TRI = Tri acetyl minocycline. *p < .05 compared to controls.

These results are consistent with the hypothesis that CMMs will lose their antimicrobial action but retain their ability to reduce ethanol consumption. CMMs are less likely to contribute to antibiotic resistance and gastrointestinal side effects with long term use. De-amino-diacetyl-minocycline significantly reduced ethanol consumption both by intraperitoneal and oral administration, and therefore emerged as a potential lead compound.

Example 2. Targeted Carbon Chain Length Modifications Alter Minocycline Therapeutic Properties to Reduce Alcohol Consumption.

Alcohol Use Disorder (AUD) is a chronic relapsing brain disease characterized by compulsive alcohol use, loss of control over alcohol intake, and a negative emotional state when not using. Minocycline has been shown to have anti-inflammatory action and considerable preclinical studies indicate its off-target properties may be useful in treating numerous diseases. The inventors have previously shown that minocycline reduced high alcohol consumption.

However, therapeutic benefits of minocycline in non-infectious diseases may be limited due to its strong antibiotic effects on healthy gut microbiota. The present inventors created, verified and tested chemically modified minocycline (CMM) analogs with ether carbon chain additions. The modifications were C1-4, or methyl-, ethyl-, propyl-, and butyl-ether addition on the -OH of the phenolic carbon ring.

Purity and molecular formula verification of the CMM analogs were done using High Performance Liquid Chromatography (HPLC) Nuclear Magnetic Resonance spectroscopy (NMR) and Liquid Chromatography-Mass Spectrometry (LC-MS) after initial identification via Thin-layer Chromatography (TLC). Zone of inhibition (ZOI) and colony forming unit (CFU) assays were used to assess the antibacterial efficacy of our CMM analogs in E. coli cell culture. Drinking-in-the-Dark (DID) and Two-Bottle Choice (2BC) procedures were utilized in both female and male C57BL/6J mice to evaluate ethanol and water consumption. The series of 4 CMM analogs were found to have increasing loss of antimicrobial action and reduction of ethanol consumption with increase of chain length. Butyl ether minocycline may be used for an AUD treatment.

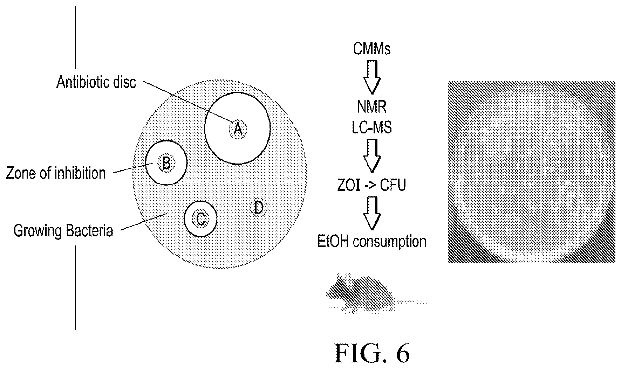

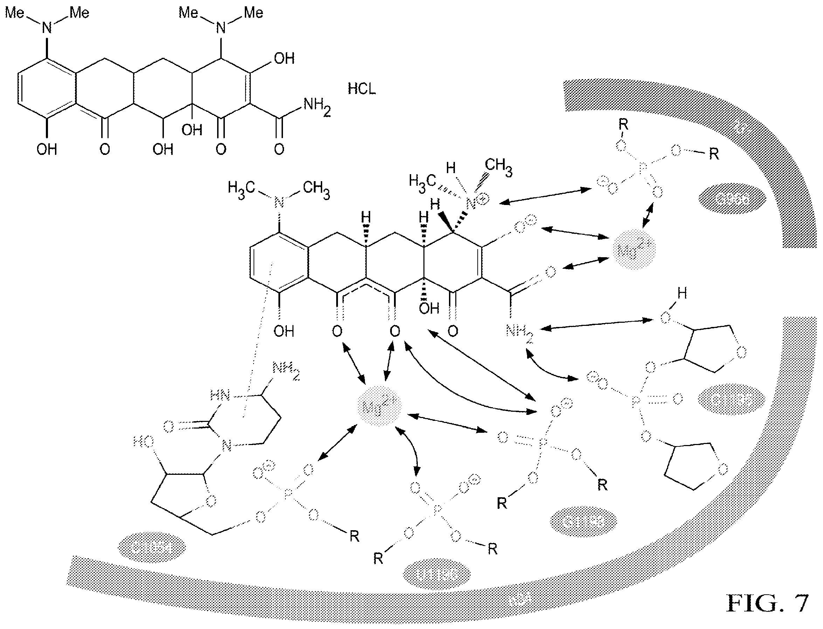

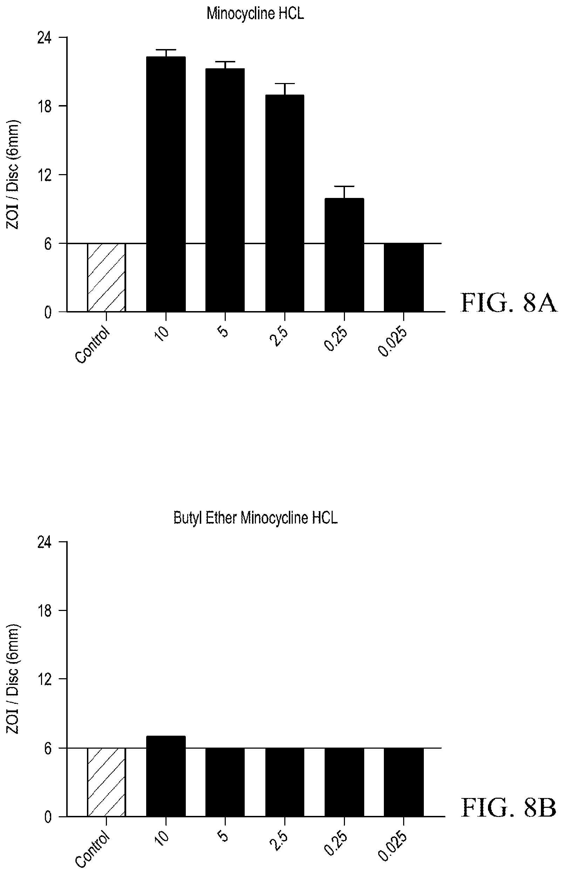

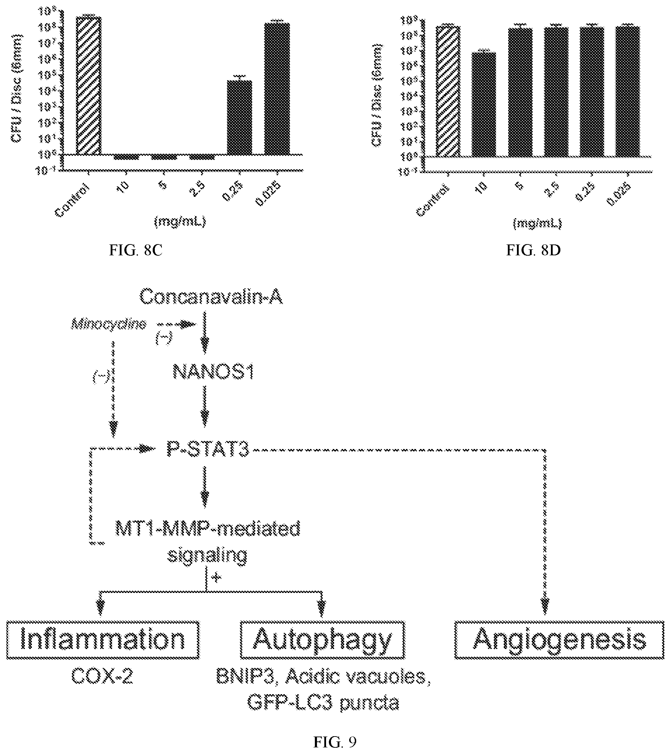

FIG. 6 shows the method of screening modifications to minocycline, CMM analogs were confirmed for purity structure utilizing HPLC, NMR and LC-MS. ZOI and CFU assays were to confirm loss of antibiotic activity, and alcohol consumption was tested in C67BL/6J mice. FIG. 7 shows the site of chemical modification on minocycline, and for reference, the solved structure of ribosome with minocycline. Schedlbauer et al., Antimicrobial Agents and Chemotherapy. 59(5): 2849-2854, 2015. FIG. 8A to 8D shows the effect of Butyl ether minocycline lost antibiotic activity compared to minocycline.

Table 3 shows the drinking results showed that butyl ether minocycline (100 mg/kg, i.p.) significantly reduced alcohol consumption.

| **Commercial Name** | **Chemical Name** | **Structure** | **Anti-microbial action** | **% DID** |
|---|---|---|---|---|
| Minocycline HCl | 4,7-Bis-dimethylamimo-3,10,12,12a-tetrahydroxy-1,11-dioxo-1,4,4a,5,5a,6,11,12a-octahydro-naphthacene-2-carboxylic acid amide hydrochloride | 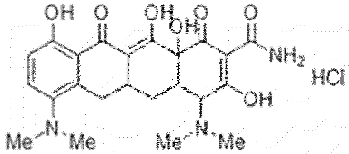 | > 0.125 mg/ml | ↓75% |
| | | | | *p* < 0.001 |
| Methyl Ether Minocycline HCl | 4,7-Bis-dimethylamino-3,12,12a-trihydroxy-10-methoxy-1,11-dioxo-1,4,4a,5,5a,6,11,12a-octahydro-naphthacene-2-carboxylic acid amide hydrochloride | 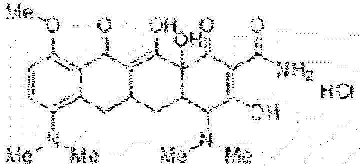 | > 16 mg/ml | ↓40% |
| | | | | *p > 0.2* |
| Ethyl Ether Minocycline HCl | 4,7-Bis-dimethylamino-10-ethoxy-3,12,12a-trihydroxy-1,11-dioxo-1,4,4a,5,5a,6,11,12a-octahydro-naphthacene-2-carboxylic acid amide hydrochloride | 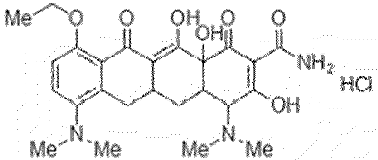 | > 2 mg/ml | ↓50% |
| | | | | *p* = 0.05 |
| Propyl Ether Minocycline HCl | 4,7-Bis-dimethylamino-3,12,12a-rihydroxy-1,11-dioxo-10-propoxy-1,4,4a,5,5a,6,11,12a-octahydro-naphthacene-2-carboxylic acid amide hydrochloride | 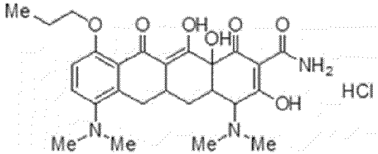 | > 4 mg/ml | ↓50% |
| | | | | *p* > 0.2 |
| Butyl Ether Minocycline HCl | 10-Butoxy-4,7-bis-damethylamino-3,12,12a-tnhydroxy-1,11-dioxo-1,4,4a,5,5a,6,11,12a-octahydronaphthacene-2-carboxylic acid amide hydrochloride | 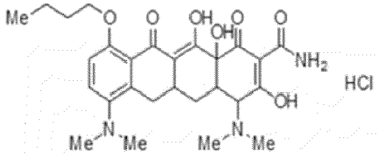 | ND 64 mg/ml | ↓85% |
| | | | | *p* < 0.0001 |

This example shows the chemical modification of minocycline to add a carbon chain on the phenolic -OH appeared to prevent bacterial ribosome binding and led to the successful reduction of relevant antibiotic activity. Of the four compounds tested, all compounds showed a decrease in alcohol consumption, with butyl ether minocycline showing the greatest reduction in alcohol consumption.

In this example, length of carbon chain was correlated with decrease in alcohol consumption suggesting that the chemical addition is not only related to loss of antimicrobial action, but may also have anti-inflammatory or other unknown positive mechanism of action (on drinking).

Example 4. Chemically modified minocycline as a novel therapeutic for rheumatoid arthritis.

Rheumatoid arthritis (RA) is a chronic inflammatory joint disorder that affects ~1% of the population. There is no cure, but several drugs promote remission. All have strong side-effects. Known anti-inflammatory, immunomodulatory, and chondro-protective properties of minocycline are posited to play a key role in RA treatment. However, an obstacle for the long-term is its negative side-effects on the gastrointestinal microbiota. The present inventors have made new chemically modified minocycline (CMM) analogs, which removed the antibiotic activity. The CMM analogs were tested as a therapeutic in a murine model of RA and shown to significantly reduce symptoms.

FIG. 9 shows a flowchart for the mechanism of action of minocycline related to autophagy, inflammation, and angiogenesis.

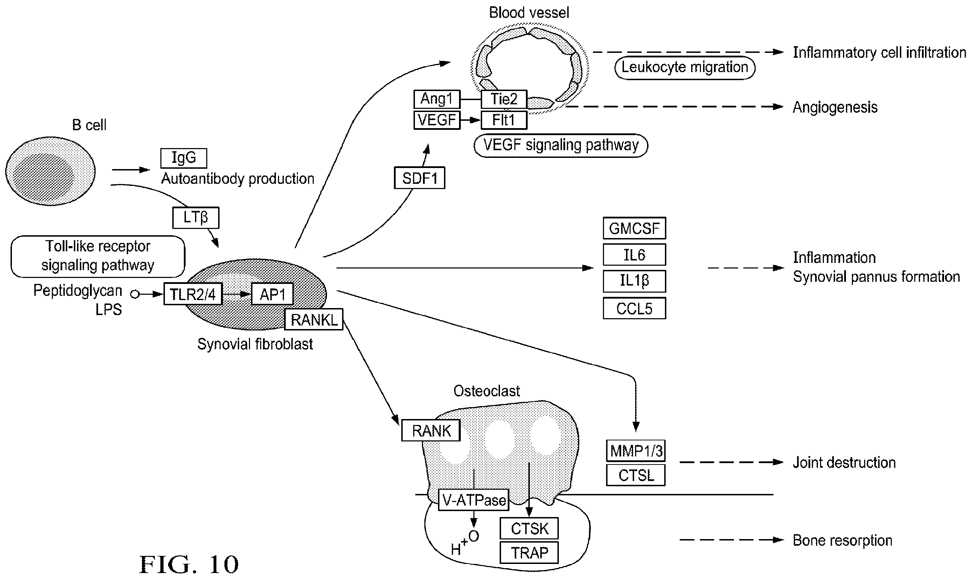

FIG. 10 shows the cellular signaling pathways for COX2, BNIP3, acidic vacuoles, and GFP-LC3 puncta.

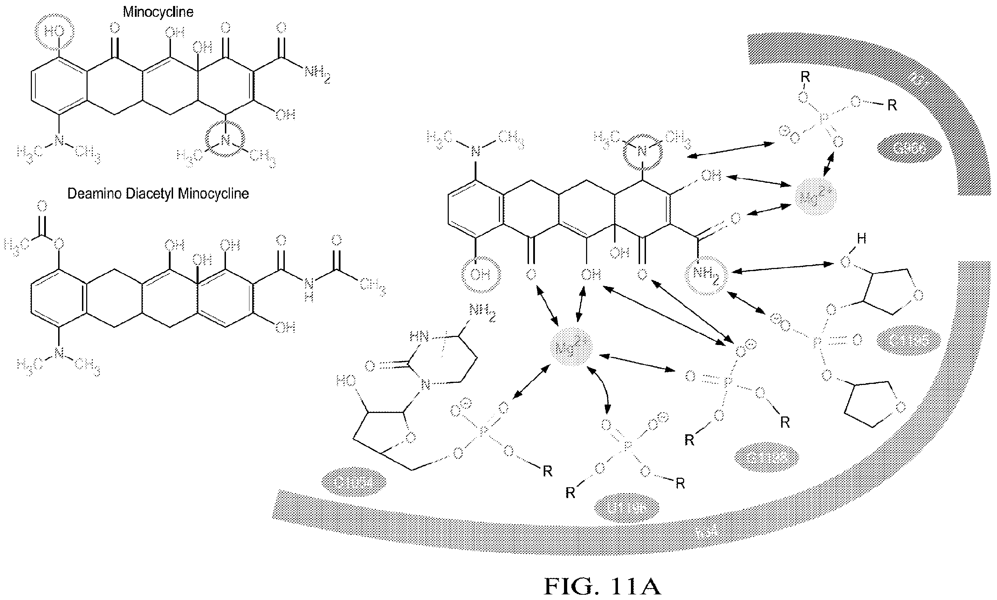

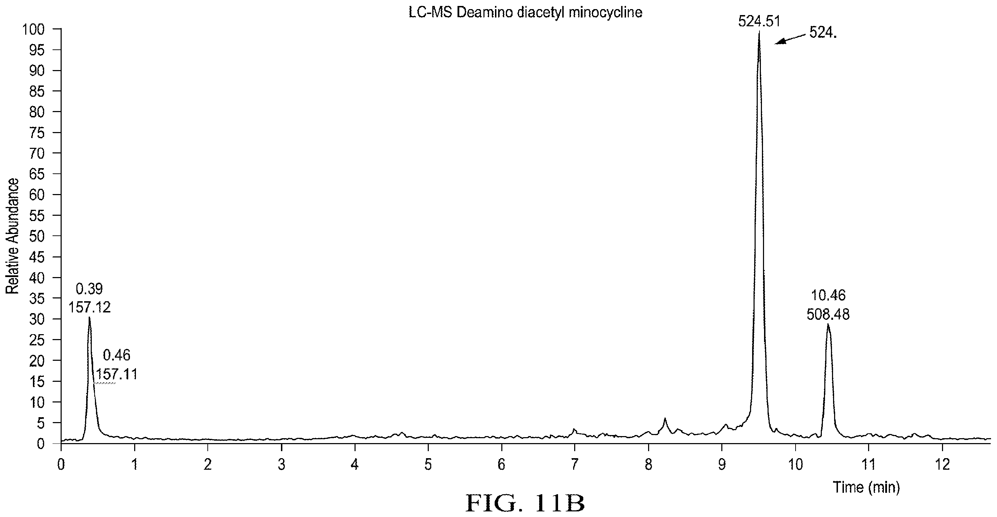

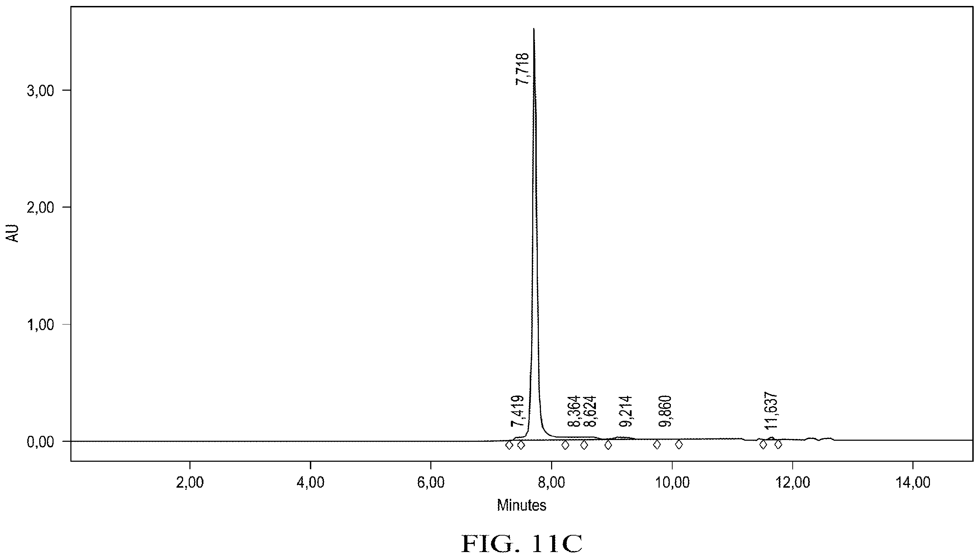

FIGS. 11A to 11C show Deamino diacetyl minocycline mass, purity, and structure were confirmed using LC-MS (FIG. 11B), and HPLC (FIG. 11C), which was further confirmed with NMR (not shown).

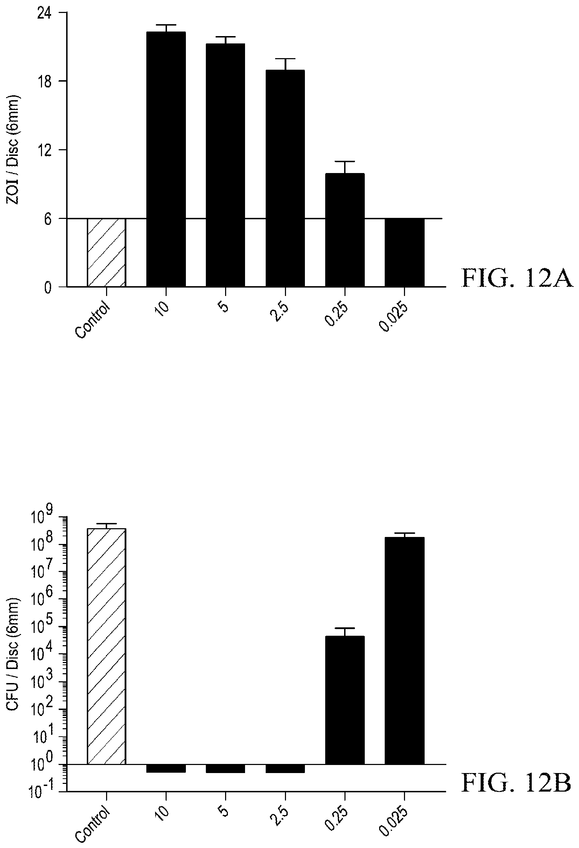

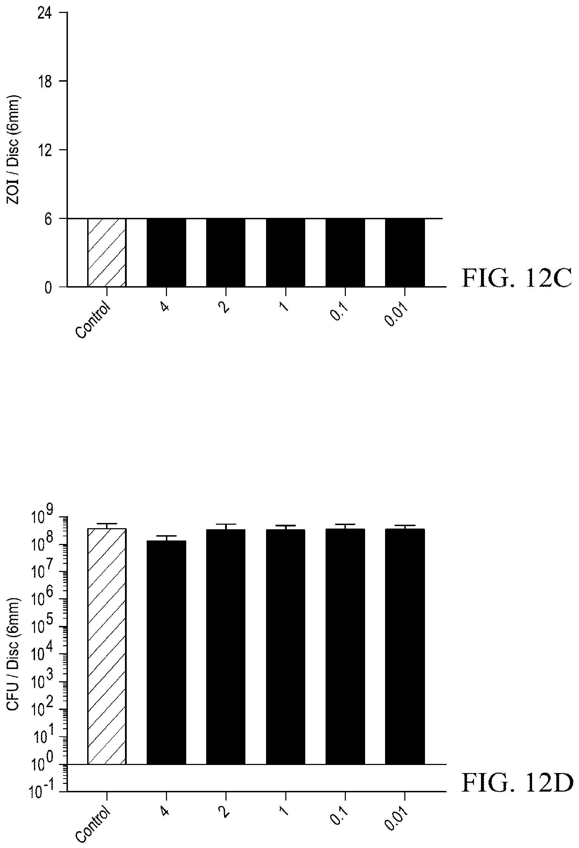

FIGS. 12A to 12D show the zone of inhibition and colony forming units assays showed loss of antibiotic activity for deamino diacetyl minocycline FIG. 12A and 12C, respectively, compared to minocycline FIGS. 12B and 12D, respectively.

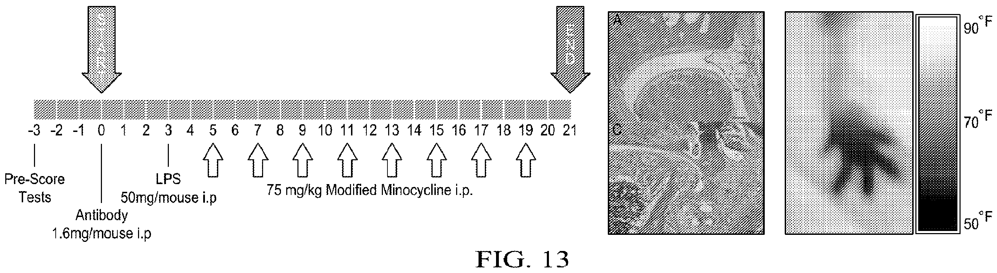

FIG. 13 shows the basic experimental set-up for the rheumatoid arthritis. The RA model was induced with 5-monoclonal antibodies directed against collagen type II followed by an E. coli lipopolysaccharide adjuvant (CIA, Chondrex, Redmond, WA) in female and male DBA/1J mice. Groups were treated with saline or 75mg/kg of CMM i.p. RA was quantified using caliper measurements, infrared heat signatures, arthritis scoring, hotplate (42°C) and Von Frey filament assays.

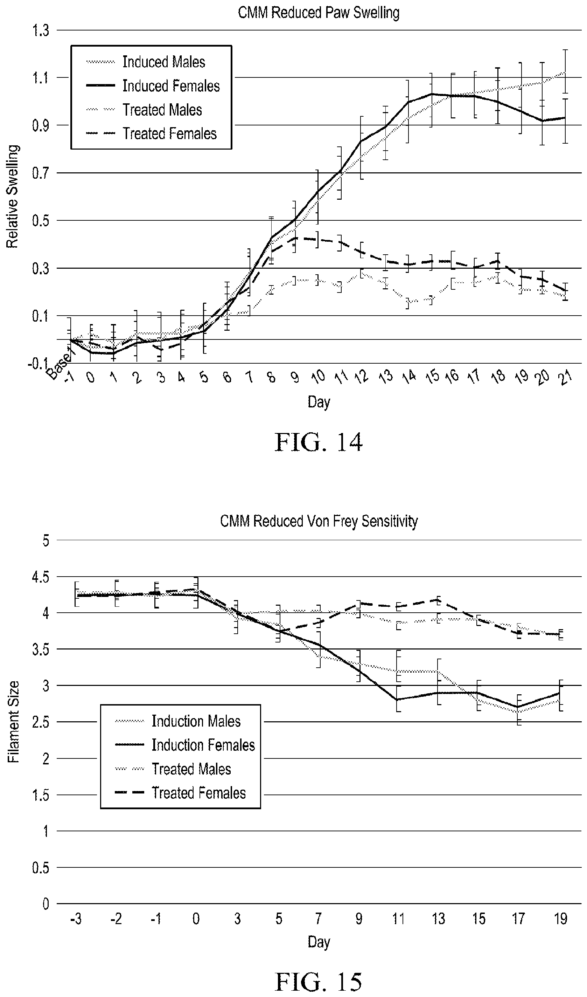

FIG. 14 shows CMM reduced paw swelling by approximately 50% as measured by caliper (mm). Date is shown as increase over baseline measurements.

FIG. 15 shows the CMM reduced pressure nociception in RA mice as measured by the von Frey filament assay. Induced mice that received only a saline treatment saw a roughly 40% increase in sensitivity after 21 days as compared to their baseline. This sensitivity was virtually eliminated from the group that received the MMC injections.

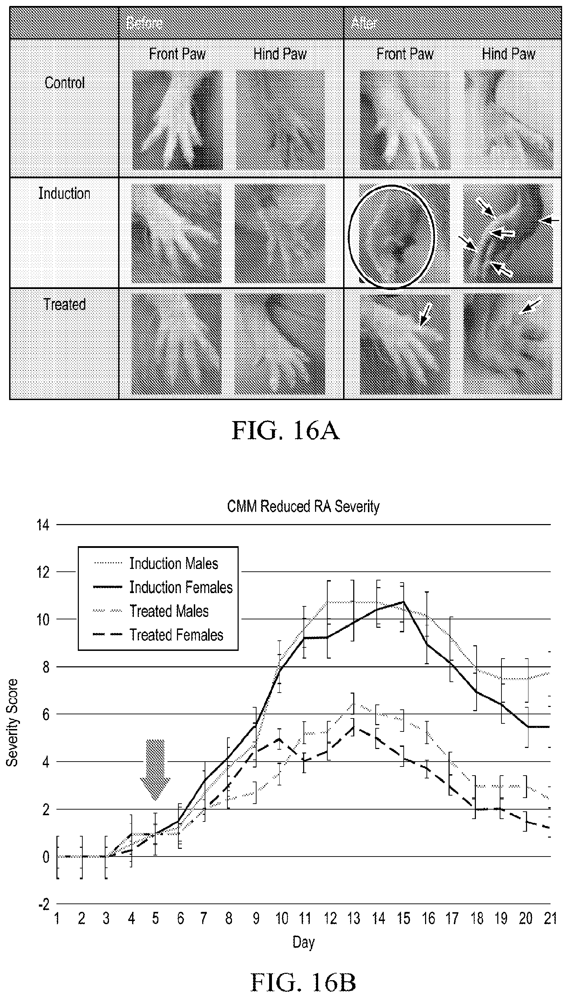

FIGS. 16A and 16B show, FIG. 16A are photos resulting from the injection with the Chondrex antibody cocktail lead to severe inflammation of all the phalangeal, metatarsal, and ankle joints along with a loss of anatomical definition in the front paw. MCC significantly reduced the RA severity with an approximately 70% reduction in arthritis scoring versus the induced group shown in FIG. 16B. Improvement was clear in both female and male mice.

Injection of Chondrex - a 5 monoclonal antibody cocktail followed by LPS adjuvant induced severe RA symptoms in the DBA/1J murine model. CMM (75mg/kg i.p. every other day) reduced the severity of RA symptoms. CMM was shown to have no antibiotic activity and, therefore, provides a long-term solution for patients with RA without affecting their gut microbiota.

Examples of salts that may be used with the compounds of the present invention include: sulfate, citrate, acetate, oxalate, chloride, bromide, iodide, nitrate, besylate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and/or pamoate.

It is contemplated that any embodiment discussed in this specification can be implemented with respect to any method, kit, reagent, or composition of the invention, and vice versa. Furthermore, compositions of the invention can be used to achieve methods of the invention.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In embodiments of any of the compositions and methods provided herein, "comprising" may be replaced with "consisting essentially of" or "consisting of". As used herein, the phrase "consisting essentially of" requires the specified integer(s) or steps as well as those that do not materially affect the character or function of the claimed invention. As used herein, the term "consisting" is used to indicate the presence of the recited integer (e.g., a feature, an element, a characteristic, a property, a method/process step or a limitation) or group of integers (e.g., feature(s), element(s), characteristic(s), property(ies), method/process steps or limitation(s)) only.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "substantial" or "substantially" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by at least ±1, 2, 3, 4, 5, 6, 7, 10, 12 or 15%.

### REFERENCES

Agrawal, R. G., A. Hewetson, C. M. George, P. J. Syapin, and S. E. Bergeson. 2011. 'Minocycline reduces ethanol drinking', Brain, behavior, and immunity, 25 Suppl 1: S165-9.

Agrawal, R. G., J. A. Owen, P. S. Levin, A. Hewetson, A. E. Berman, S. R. Franklin, R. J. Hogue, Y. Chen, C. Walz, B. D. Colvard, J. Nguyen, O. Velasquez, Y. Al-Hasan, Y. A. Blednov, A. K. Fowler, P. J. Syapin, and S. E. Bergeson. 2014. 'Bioinformatics analyses reveal age-specific neuroimmune modulation as a target for treatment of high ethanol drinking', Alcoholism, clinical and experimental research, 38: 428-37.

Bergeson, S. E., H. Blanton, J. M. Martinez, D. C. Curtis, C. Sherfey, B. Seegmiller, P. C. Marquardt, J. A. Groot, C. L. Allison, C. Bezboruah, and J. Guindon. 2016. 'Binge Ethanol Consumption Increases Inflammatory Pain Responses and Mechanical and Cold Sensitivity: Tigecycline Treatment Efficacy Shows Sex Differences', Alcoholism, clinical and experimental research, 40: 2506-15.

Bergeson, S. E., M. A. Nipper, J. Jensen, M. L. Helms, and D. A. Finn. 2016. 'Tigecycline Reduces Ethanol Intake in Dependent and Nondependent Male and Female C57BL/6J Mice', Alcoholism, clinical and experimental research, 40: 2491-98.

Blednov, Y. A., J. M. Benavidez, C. Geil, S. Perra, H. Morikawa, and R. A. Harris. 2011. 'Activation of inflammatory signaling by lipopolysaccharide produces a prolonged increase of voluntary alcohol intake in mice', Brain, behavior, and immunity, 25 Suppl 1: S92-S105.

Martinez, J. M., J. A. Groot, D. C. Curtis, C. L. Allison, P. C. Marquardt, A. N. Holmes, D. S. Edwards, D. R. Trotter, P. J. Syapin, D. A. Finn, and S. E. Bergeson. 2016. 'Effective Reduction of Acute Ethanol Withdrawal by the Tetracycline Derivative, Tigecycline, in Female and Male DBA/2J Mice', Alcoholism, clinical and experimental research, 40: 2499-505.

Montesinos, J., S. Alfonso-Loeches, and C. Guerri. 2016. 'Impact of the Innate Immune Response in the Actions of Ethanol on the Central Nervous System', Alcoholism, clinical and experimental research, 40: 2260-70.

Rhodes, J. S., K. Best, J. K. Belknap, D. A. Finn, and J. C. Crabbe. 2005. 'Evaluation of a simple model of ethanol drinking to intoxication in C57BL/6J mice', Physiology & Behavior, 84: 53-63.

Schedlbauer, A., T. Kaminishi, B. Ochoa-Lizarralde, N. Dhimole, S. Zhou, J. P. Lopez-Alonso, S. R. Connell, and P. Fucini. 2015. 'Structural characterization of an alternative mode of tigecycline binding to the bacterial ribosome', Antimicrobial agents and chemotherapy, 59: 2849-54.

Syapin, P. J., J. M. Martinez, D. C. Curtis, P. C. Marquardt, C. L. Allison, J. A. Groot, C. Baby, Y. M. Al-Hasan, I. Segura-Ulate, M. J. Scheible, K. T. Nicholson, J. L. Redondo, D. R. Trotter, D. S. Edwards, and S. E. Bergeson. 2016. 'Effective Reduction in High Ethanol Drinking by Semisynthetic Tetracycline Derivatives', Alcoholism, clinical and experimental research, 40: 2482-90.

## Claims

1. A tetracycline molecule, pharmaceutically acceptable salts and tautomers thereof, selected from 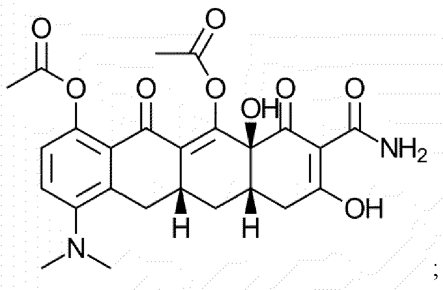 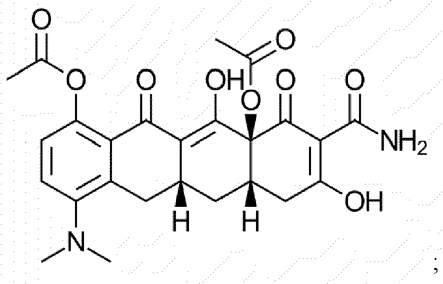 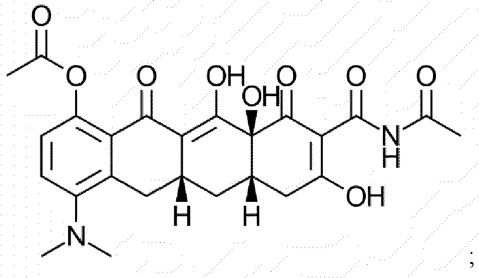 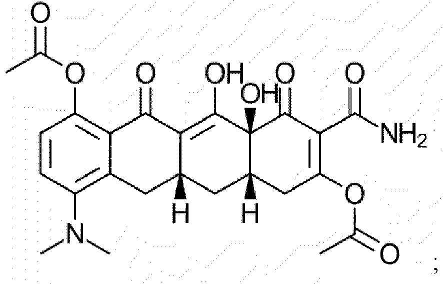 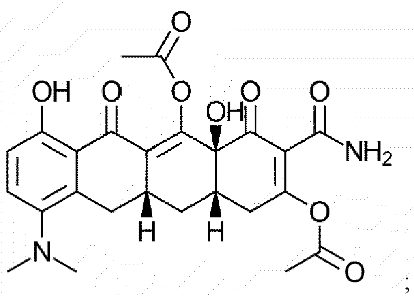 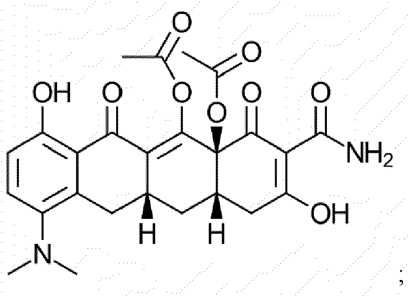 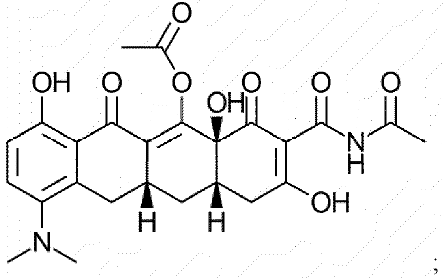 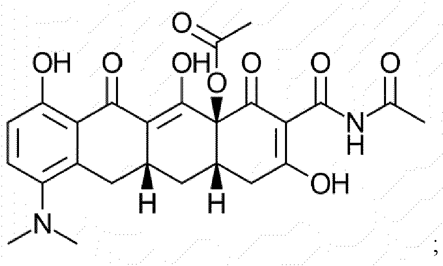 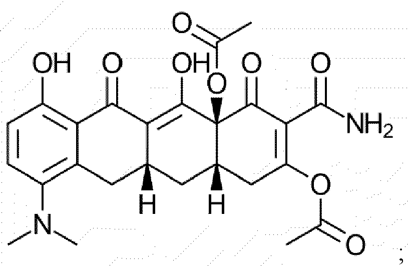 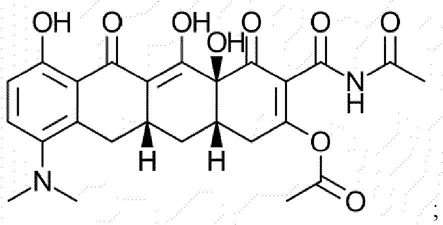 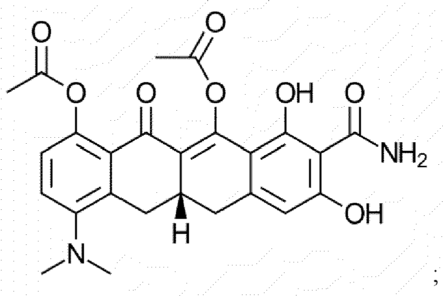 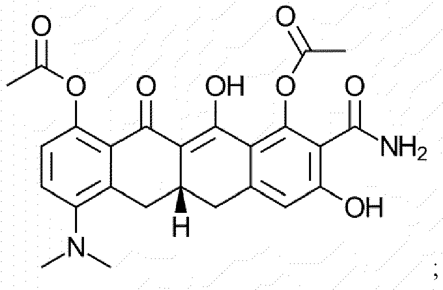 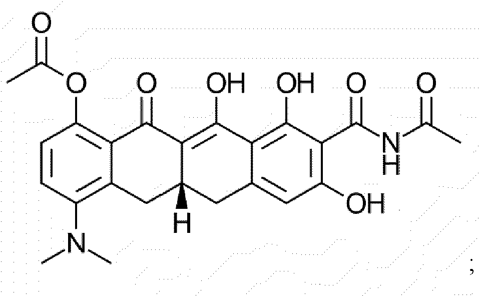 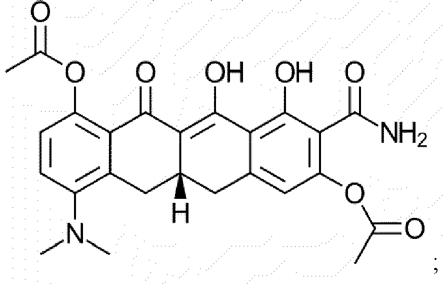 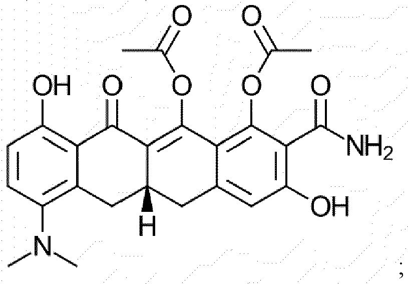 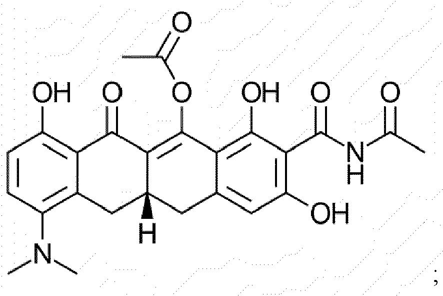 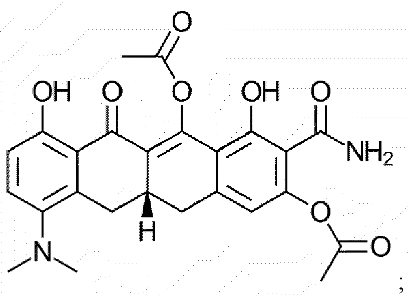 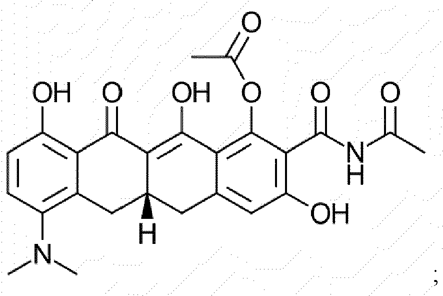 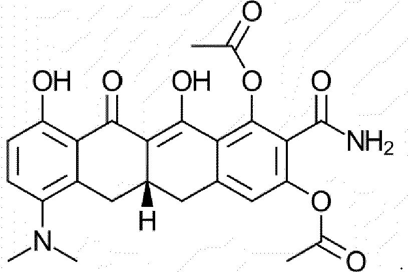 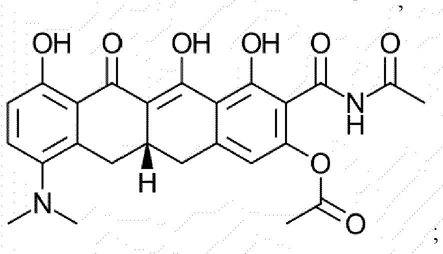 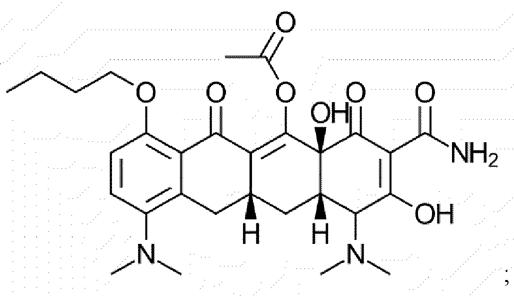 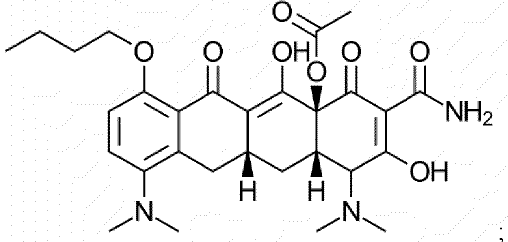 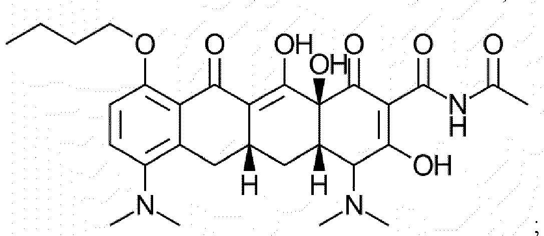 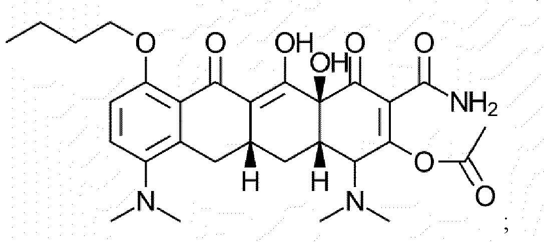 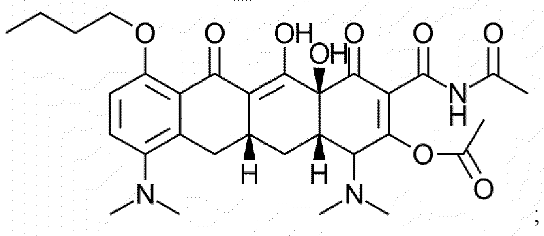 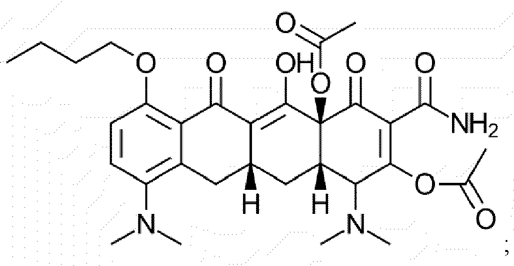 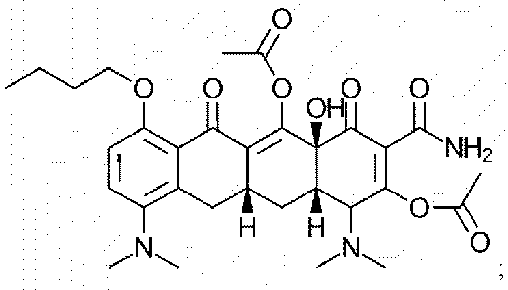 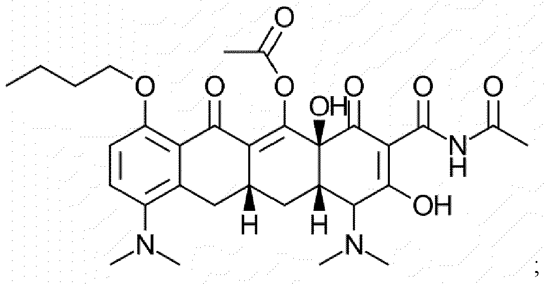 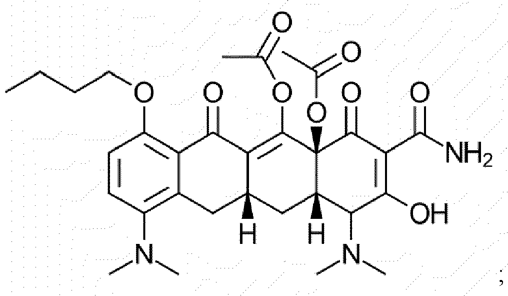 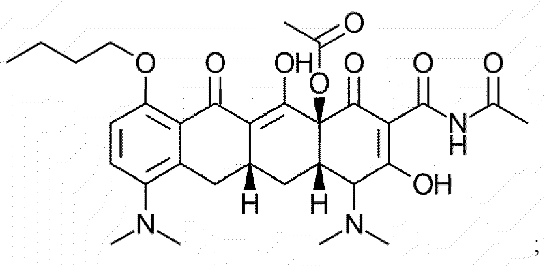 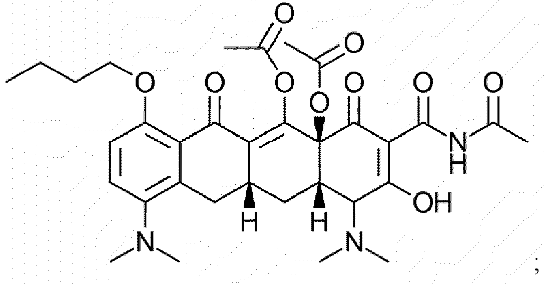 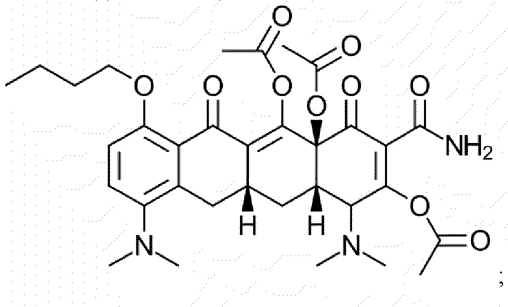 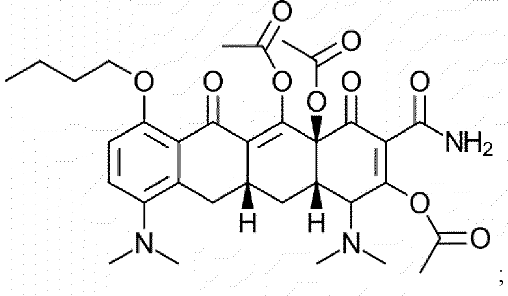 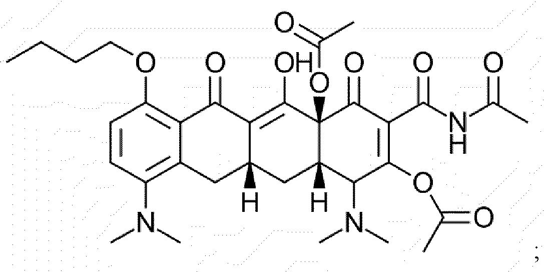 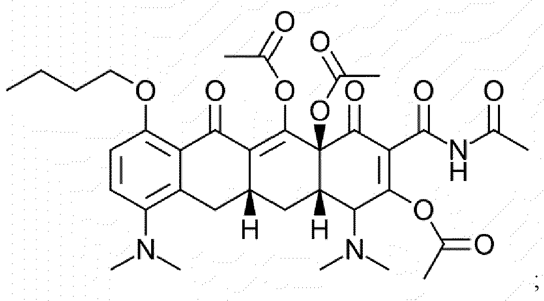 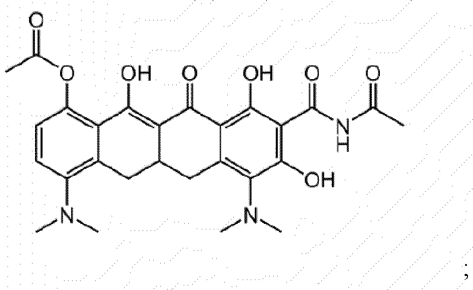 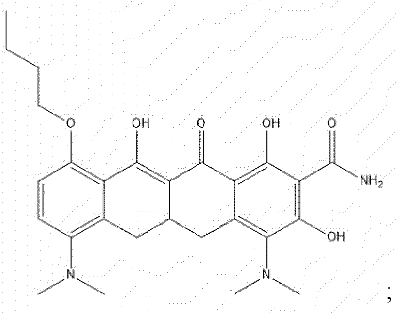 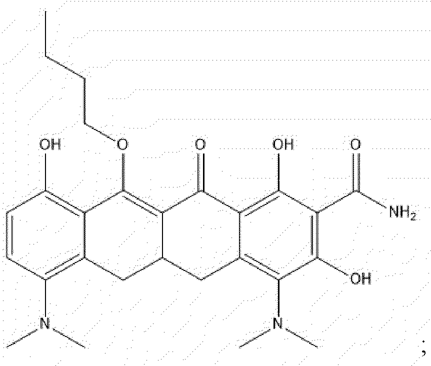 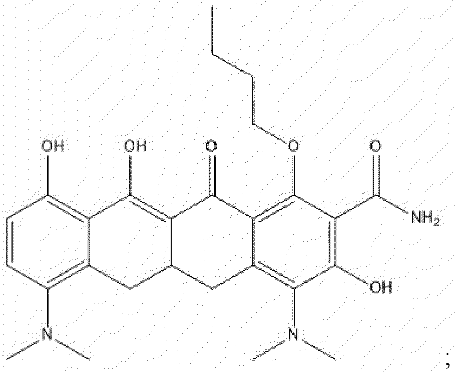 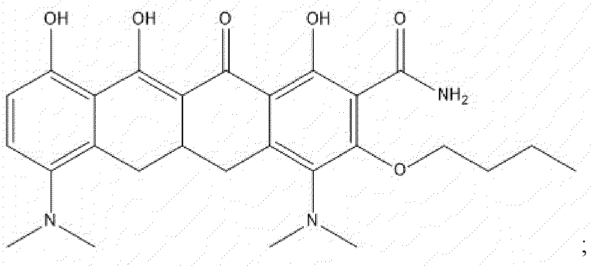 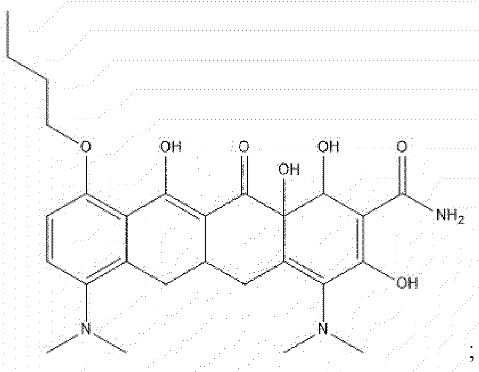 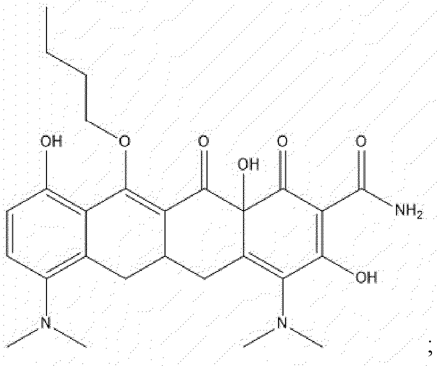 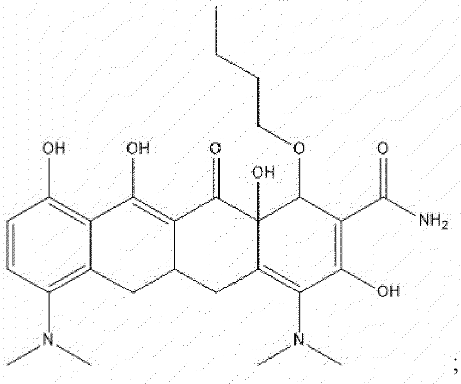 or

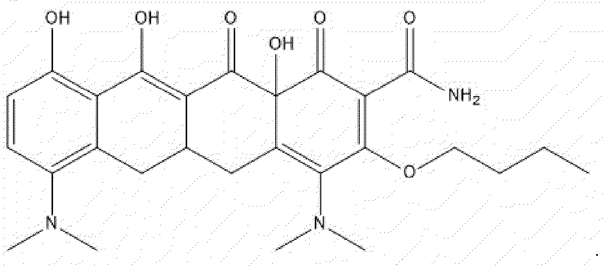

2. The molecule of claim 1, wherein the molecule has reduced or substantially no antibacterial activity, no antifungal activity, or both.

3. The molecule of any one of claims 1 or 2, wherein the molecule is disposed in a pharmaceutically acceptable buffer, excipient, filler, or carrier, or is formulated in a composition for administration orally, enterally, intramuscularly, parenterally, intravenously, or intraperitoneally.

4. A pharmaceutical composition comprising a compound of Formula **1** to **20** or **25** to **48,** pharmaceutically acceptable salts, or tautomers thereof.

5. One or more tetracycline of Formula **1** to **20** or **25** to **48** for use in the treatment of Alcohol Use Disorder (AUD), Substance Use Disorder (SUD), tobacco use, pain, a proinflammatory disorder, or rheumatoid arthritis comprising:
providing the subject with an effective amount of one or more tetracyclines of Formula 1 to **20** or **25** to **48** to ameliorate or eliminate the AUD, SUD, pain, or proinflammatory disorder.

6. The tetracycline for use according to claim 5, wherein the molecule has reduced or substantially no antibacterial activity, no antifungal activity, or both.

7. The tetracycline for use according to any one of claims 5 or 6, further comprising providing the molecule in a pharmaceutically acceptable buffer, excipient, filler, or carrier; or formulating a composition for administration orally, enterally, intramuscularly, parenterally, intravenously, or intraperitoneally.

## Patentansprüche

1. Ein Tetracyclin-Molekül, pharmazeutisch unbedenkliche Salze und Tautomere davon, ausgewählt aus 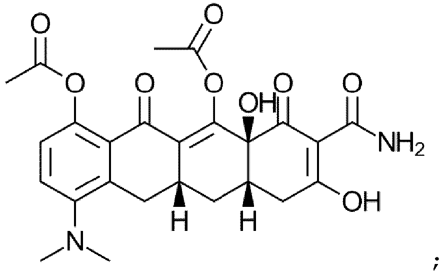 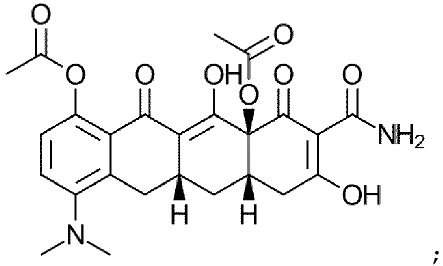 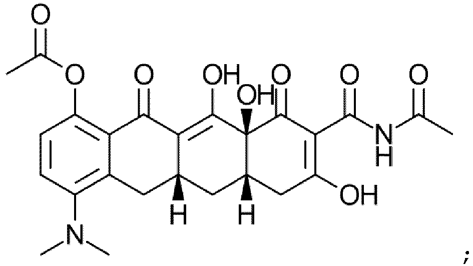 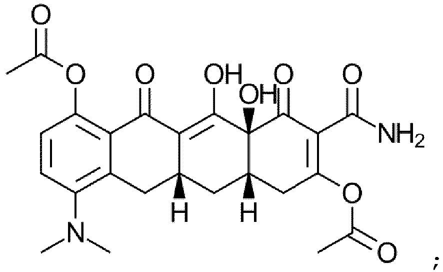 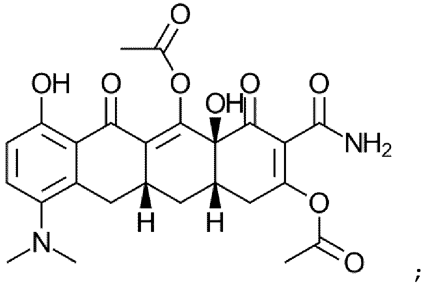 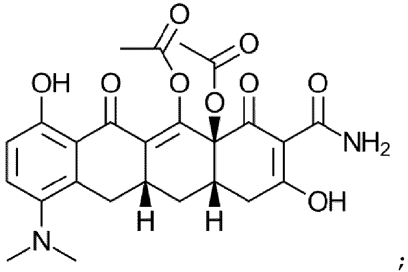 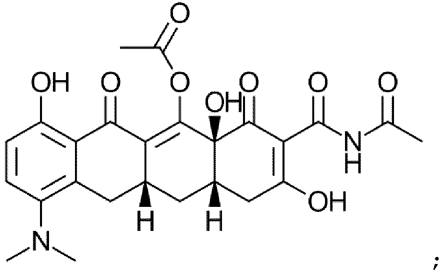 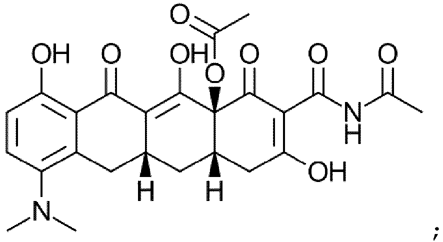 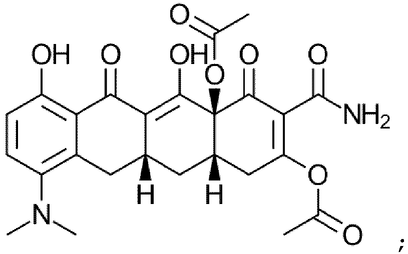 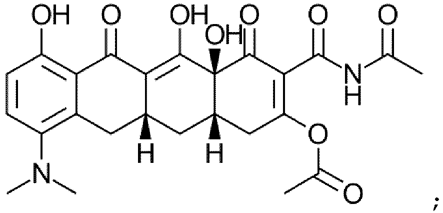 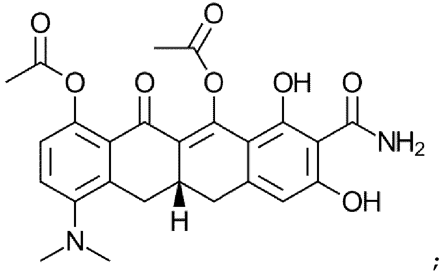 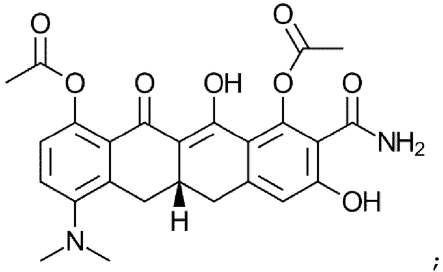 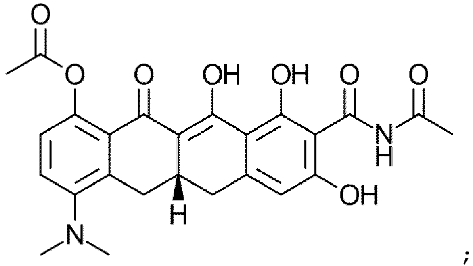 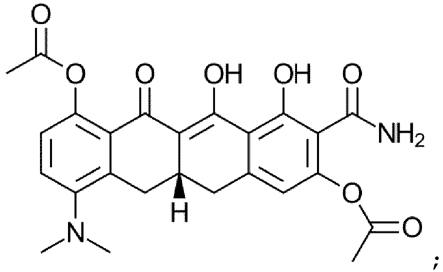 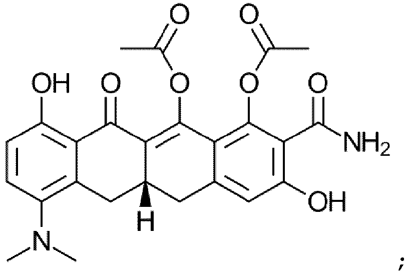 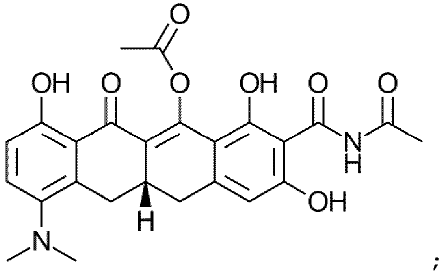 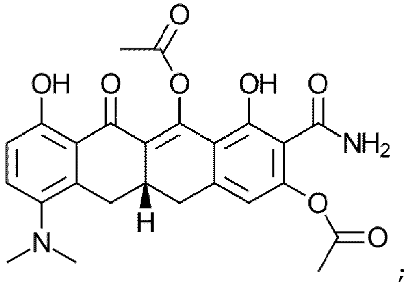 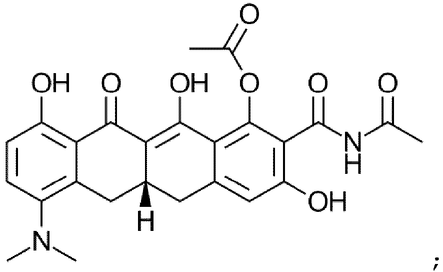 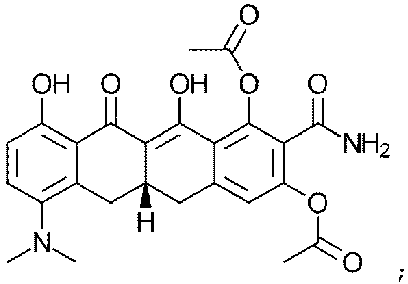 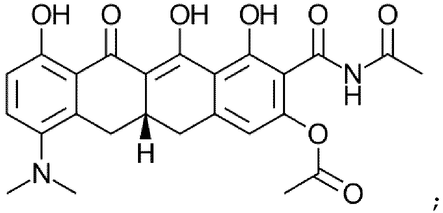 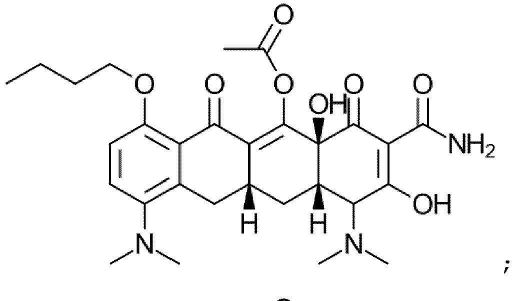 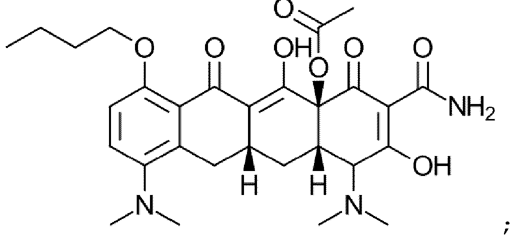 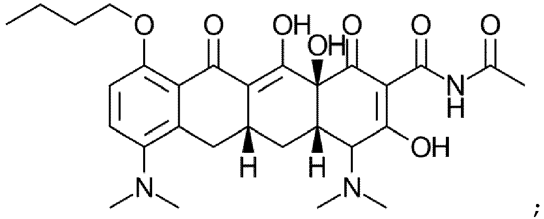 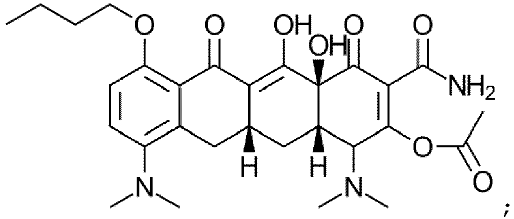 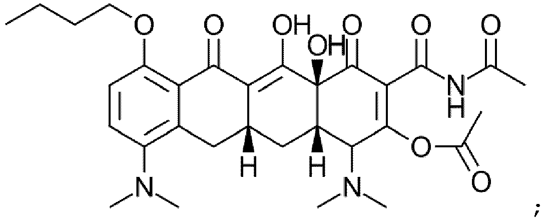 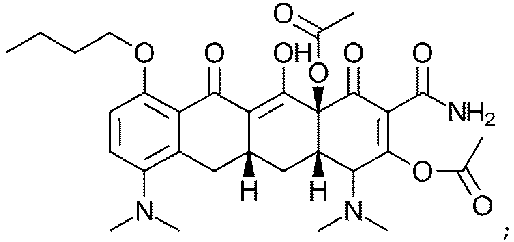 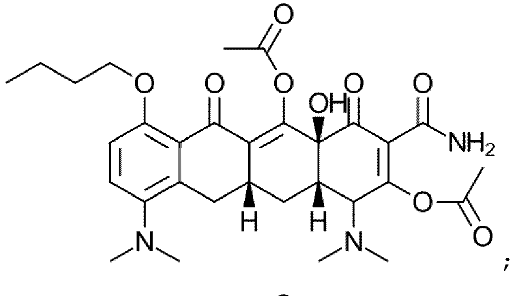 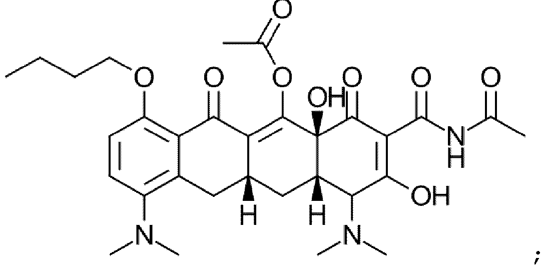 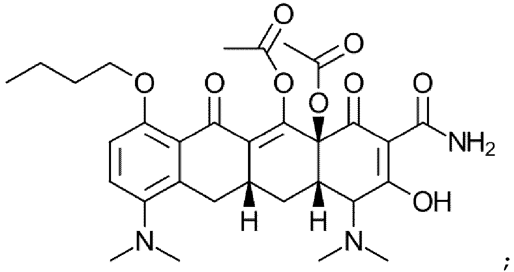 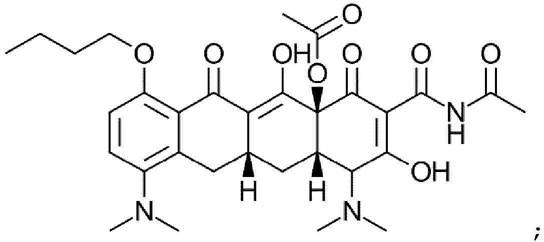 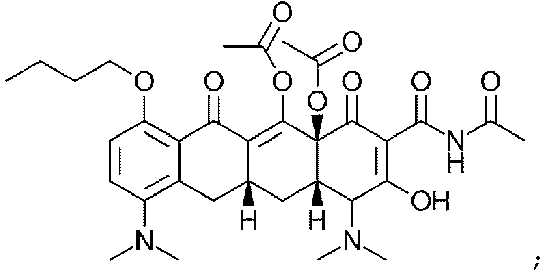 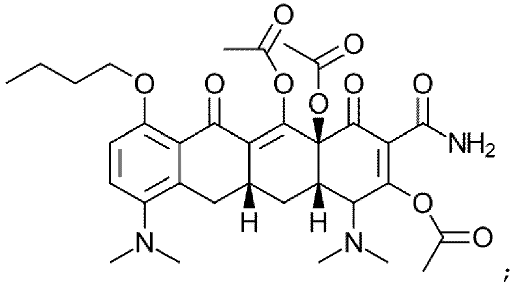 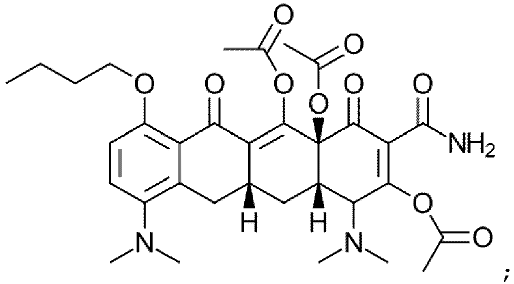 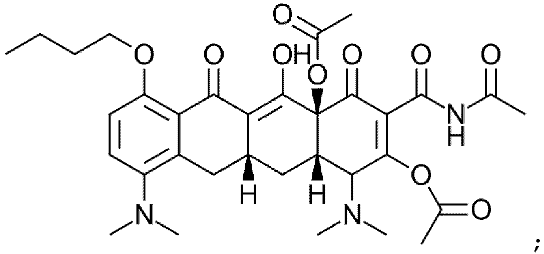 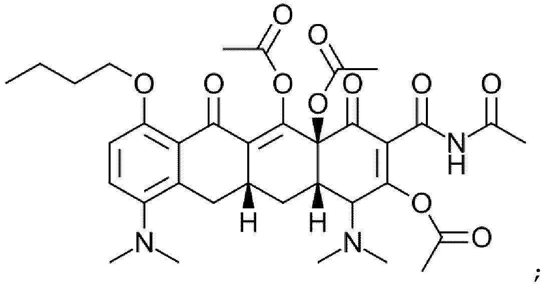 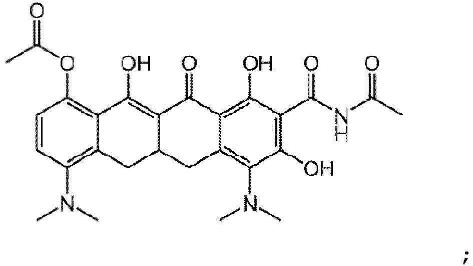 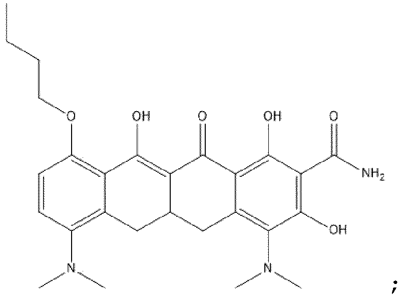 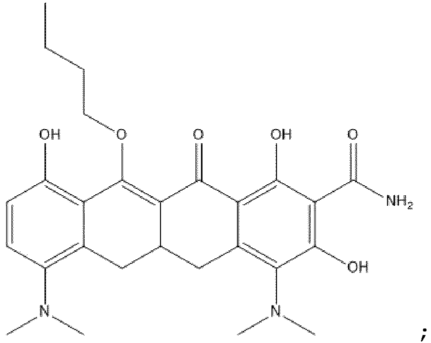 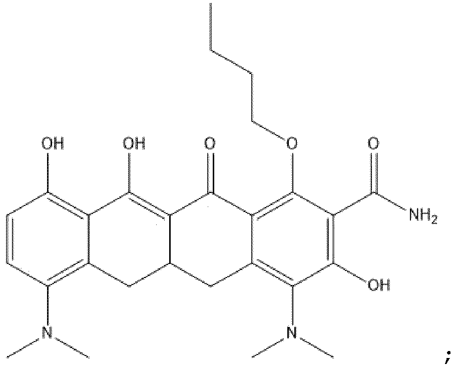 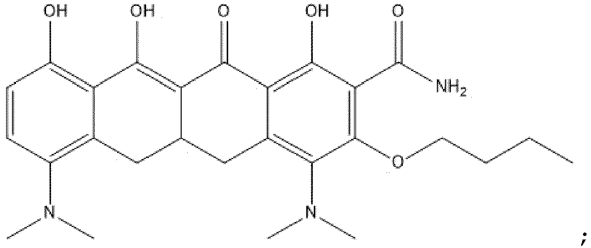 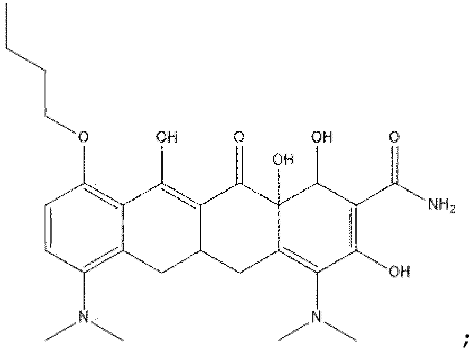 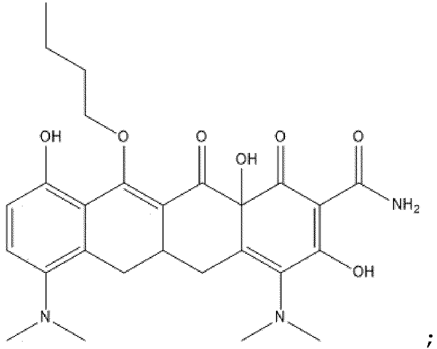 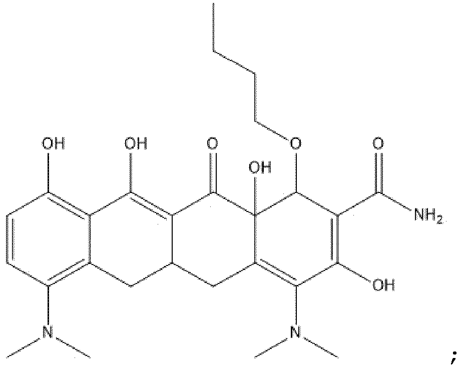 oder

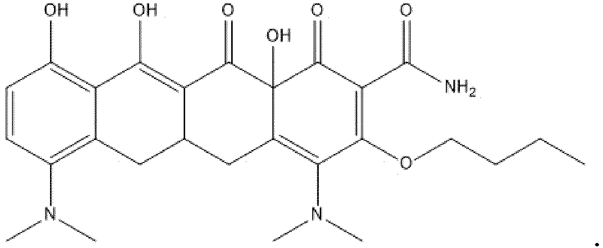

2. Das Molekül nach Anspruch 1, wobei das Molekül eine verringerte oder im Wesentlichen keine antibakterielle Aktivität, keine antifungale Aktivität oder beides aufweist.

3. Das Molekül nach einem der Ansprüche 1 oder 2, wobei das Molekül in einem pharmazeutisch unbedenklichen Puffer, Hilfsstoff, Füllstoff oder Träger vorliegt oder in einer Zusammensetzung zur oralen, enteralen, intramuskulären, parenteralen, intravenösen oder intraperitonealen Verabreichung formuliert ist.

4. Eine pharmazeutische Zusammensetzung, umfassend eine Verbindung der Formel 1 bis 20 oder 25 bis 48, pharmazeutisch unbedenkliche Salze oder Tautomere davon.

5. Ein oder mehrere Tetracycline der Formel 1 bis 20 oder 25 bis 48 zur Verwendung bei der Behandlung von Alkoholkonsumstörung (Alcohol Use Disorder, AUD), Substanzkonsumstörung (Substance Use Disorder, SUD), Tabakkonsum, Schmerzen, einer proinflammatorischen Störung oder rheumatoider Arthritis, umfassend:
Bereitstellen einer wirksamen Menge eines oder mehrerer Tetracycline der Formel 1 bis 20 oder 25 bis 48 an das Subjekt, um die AUD, SUD, Schmerzen oder proinflammatorische Störung zu lindern oder zu beseitigen.

6. Das Tetracyclin zur Verwendung nach Anspruch 5, wobei das Molekül eine verringerte oder im Wesentlichen keine antibakterielle Aktivität, keine antifungale Aktivität oder beides aufweist.

7. Das Tetracyclin zur Verwendung nach einem der Ansprüche 5 oder 6, ferner umfassend das Bereitstellen des Moleküls in einem pharmazeutisch unbedenklichen Puffer, Hilfsstoff, Füllstoff oder Träger; oder das Formulieren einer Zusammensetzung zur oralen, enteralen, intramuskulären, parenteralen, intravenösen oder intraperitonealen Verabreichung.

## Revendications

1. Une molécule de tétracycline, ses sels pharmaceutiquement acceptables et ses tautomères, choisie parmi 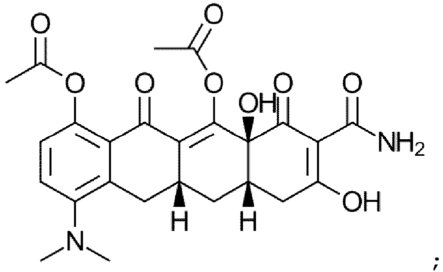 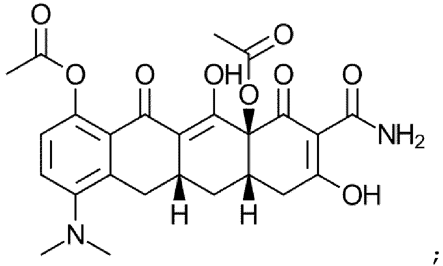 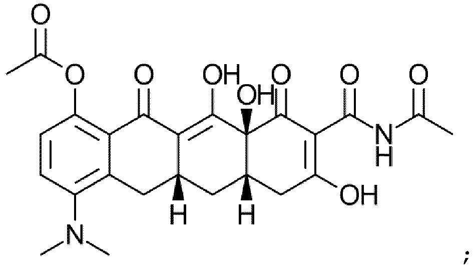 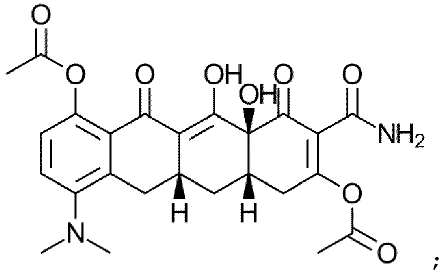 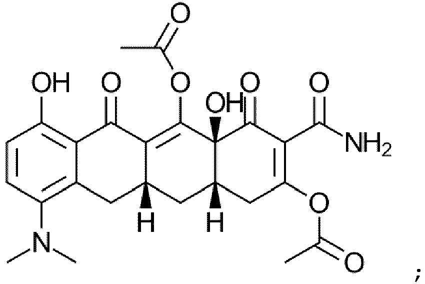 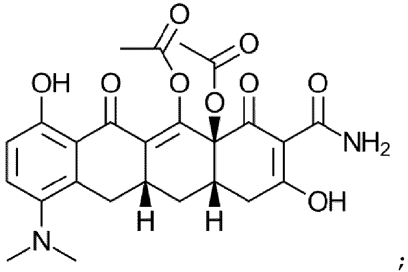 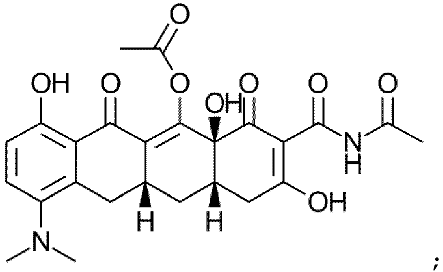 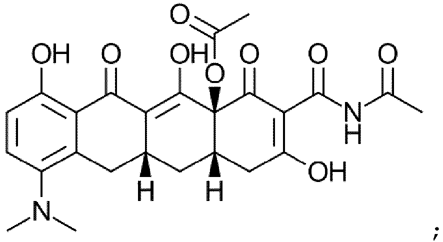 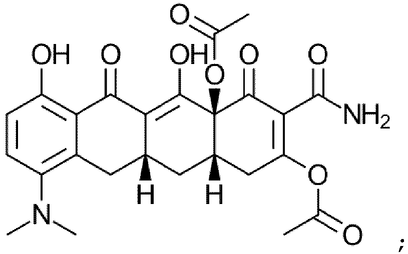 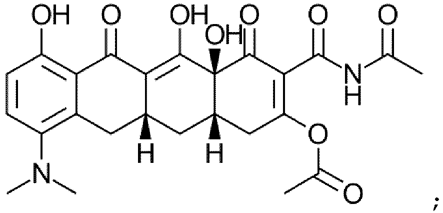 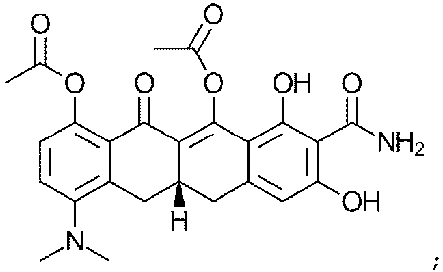 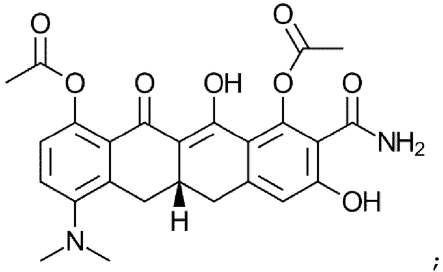 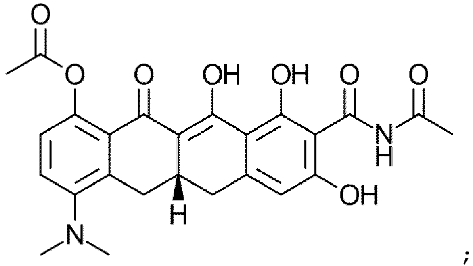 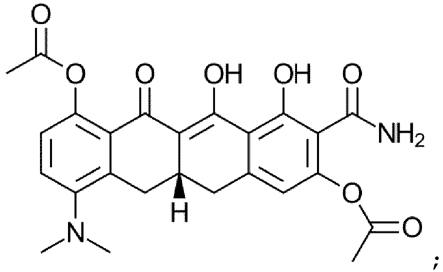 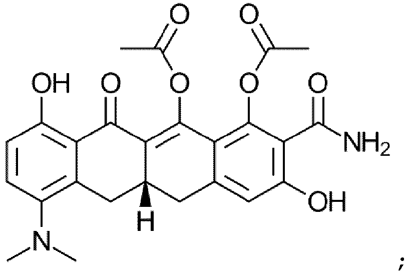 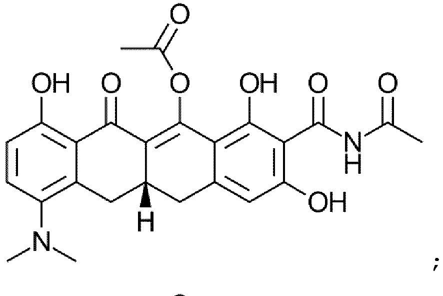 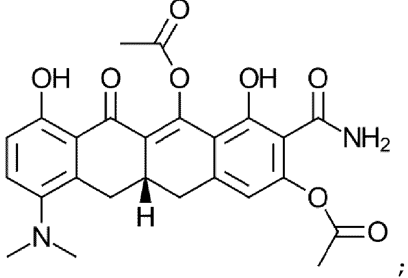 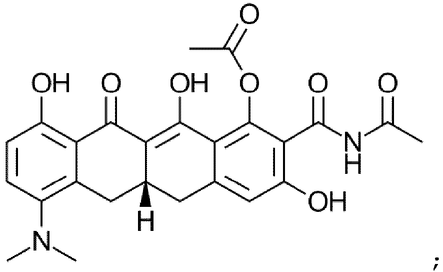 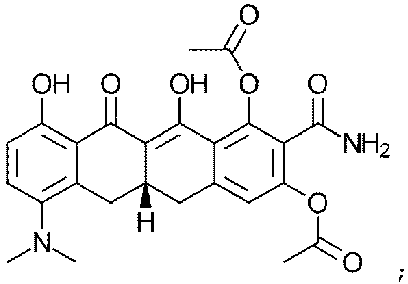 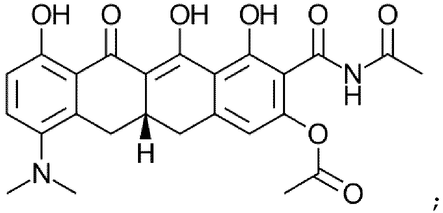 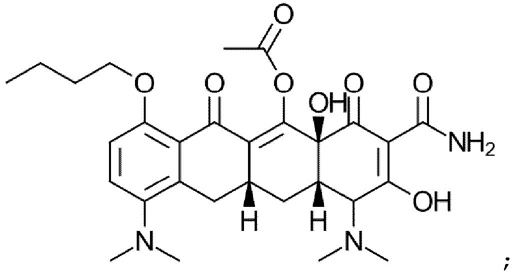 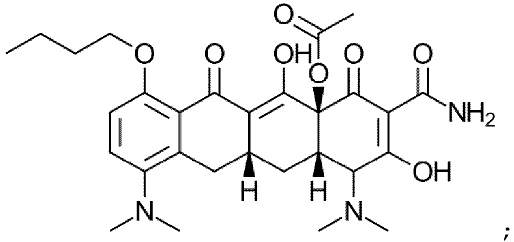 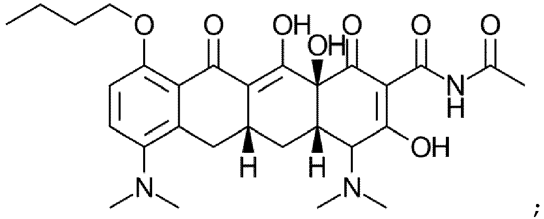 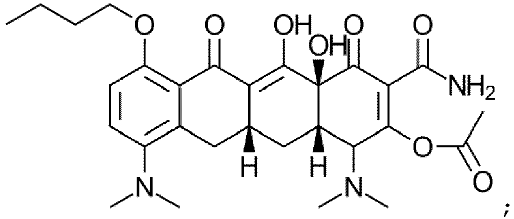 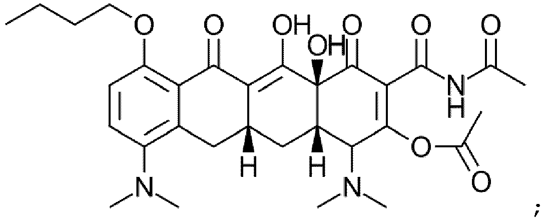 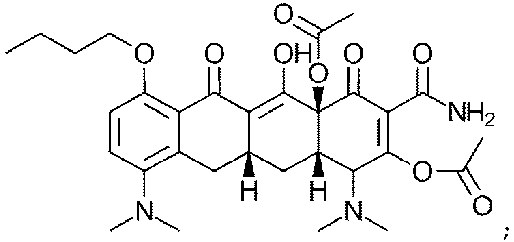 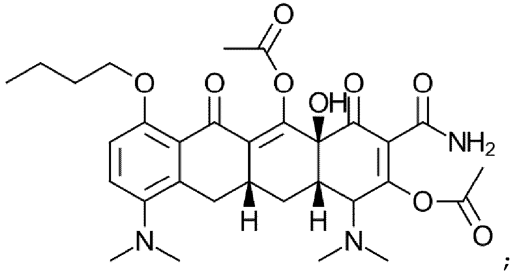 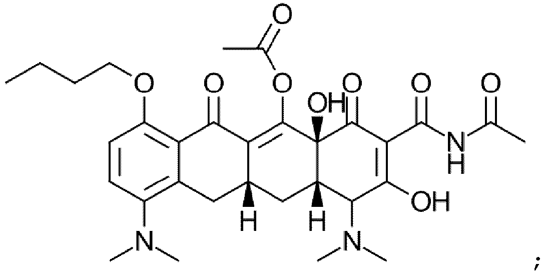 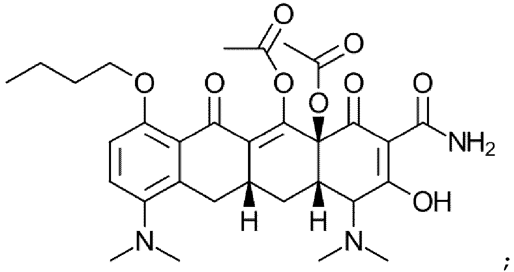 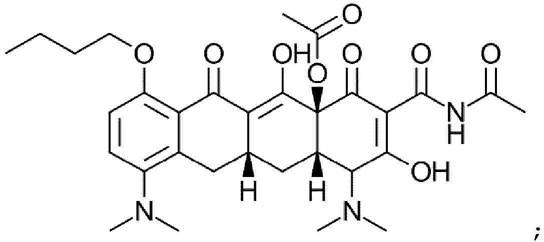 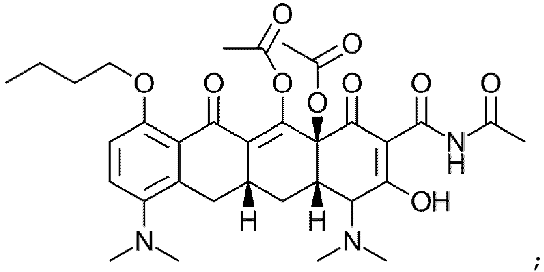 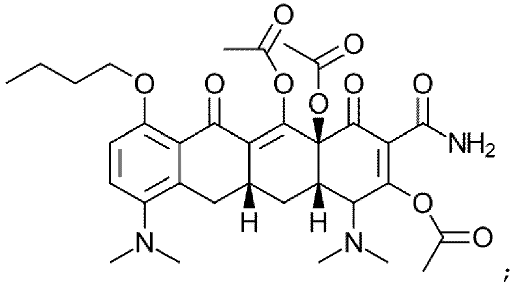 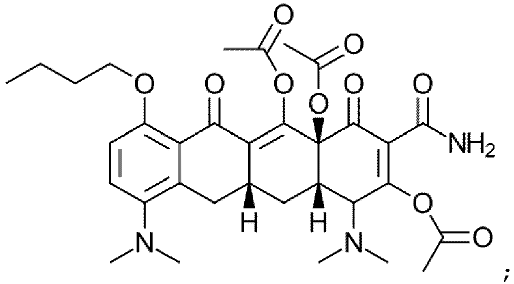 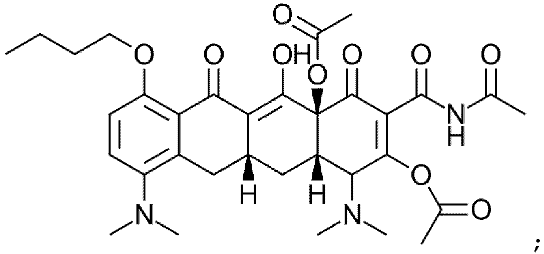 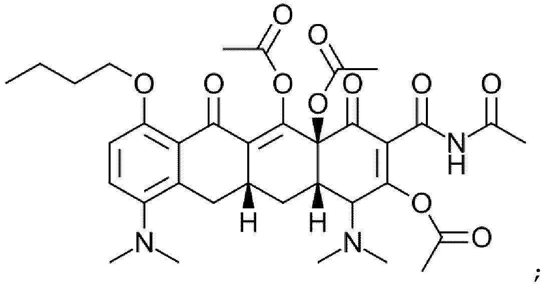 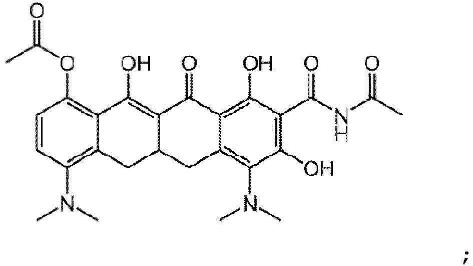 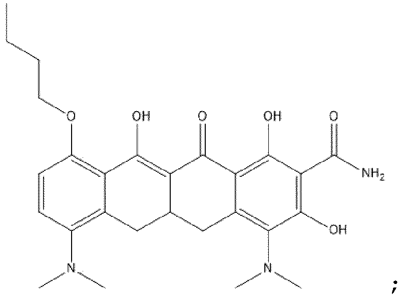 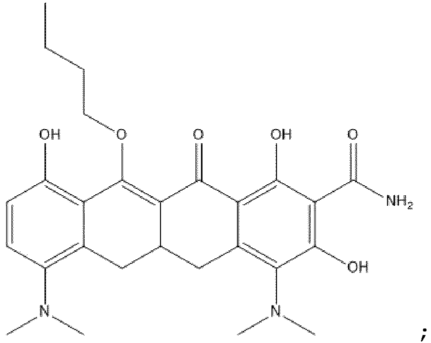 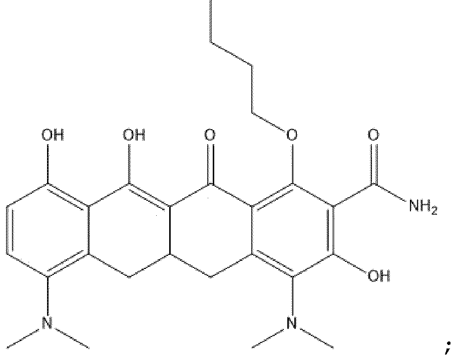 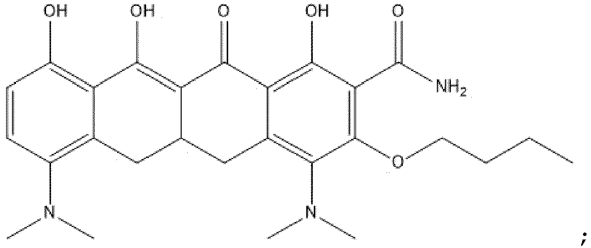 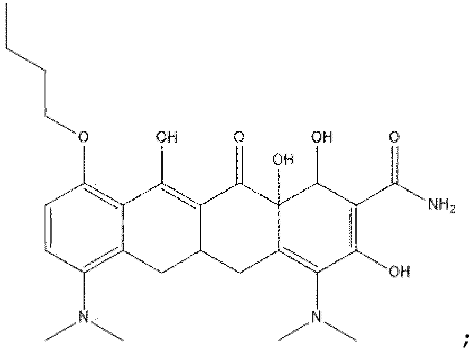 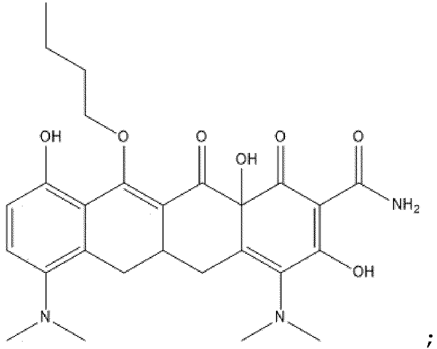 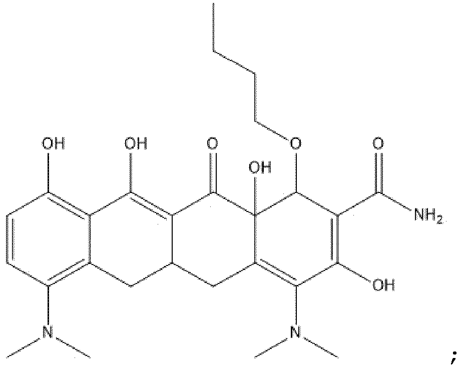 ou

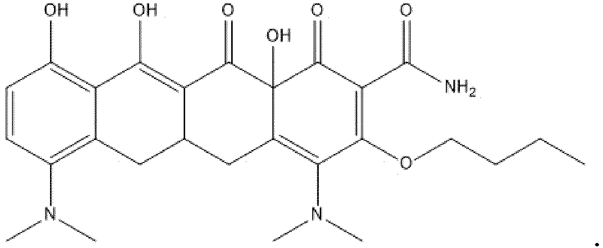

2. La molécule selon la revendication 1, dans laquelle la molécule présente une activité antibactérienne réduite ou substantiellement absente, une activité antifongique réduite ou substantiellement absente, ou les deux.

3. La molécule selon l'une quelconque des revendications 1 ou 2, dans laquelle la molécule est présente dans un tampon, un excipient, une charge ou un support pharmaceutiquement acceptable, ou est formulée dans une composition destinée à l'administration orale, entérale, intramusculaire, parentérale, intraveineuse ou intrapéritonéale.

4. Composition pharmaceutique comprenant un composé de formule 1 à 20 ou 25 à 48, ses sels pharmaceutiquement acceptables ou ses tautomères.

5. Une ou plusieurs tétracyclines de formule 1 à 20 ou 25 à 48 pour une utilisation dans le traitement du trouble de l'usage de l'alcool (Alcohol Use Disorder, AUD), du trouble de l'usage de substances (Substance Use Disorder, SUD), de la consommation de tabac, de la douleur, d'un trouble pro-inflammatoire ou de la polyarthrite rhumatoïde, comprenant:
l'administration au sujet d'une quantité efficace d'une ou plusieurs tétracyclines de formule 1 à 20 ou 25 à 48 afin d'atténuer ou d'éliminer l'AUD, le SUD, la douleur ou le trouble pro-inflammatoire.

6. La tétracycline pour une utilisation selon la revendication 5, dans laquelle la molécule présente une activité antibactérienne réduite ou substantiellement absente, une activité antifongique réduite ou substantiellement absente, ou les deux.

7. La tétracycline pour une utilisation selon l'une quelconque des revendications 5 ou 6, comprenant en outre la fourniture de la molécule dans un tampon, un excipient, une charge ou un support pharmaceutiquement acceptable ; ou la formulation d'une composition destinée à l'administration orale, entérale, intramusculaire, parentérale, intraveineuse ou intrapéritonéale.
